# DEMANDE DE BREVET EUROPEEN

(11) **EP 4 775 196 A1**
(43) Date de publication de la demande: **15.07.2026**
(21) Numéro de dépôt: 25223935.5
(22) Date de dépôt: 16.12.2025
(51) Int. Cl.: A61K 8/04, A61K 8/81, A61Q 19/00, C08F 224/00, C08F 2/06, C08F 2/10, C08F 222/02, A61K 8/06, C08F 2/18, A61K 47/34, A61K 47/32, A61K 9/00

(54) **NOUVEAUX COPOLYMERES; PROCEDE POUR LEUR PREPARATION ; UTILISATION EN COSMETIQUE ; FORMULATIONS COSMETIQUES EN COMPRENANT**

(30) Priorité: 08.01.2025 FR 2500147
(71) Demandeur: Société d'Exploitation de Produits pour les Industries Chimiques SEPPIC, 75321 Paris cedex 07 (FR)
(72) Inventeur: MONTEILLET, Stéphane, 75321 Paris Cedex 07 (FR); BODOC, Miruna, 75321 Paris Cedex 07 (FR)
(74) Mandataire: Air Liquide

(57) **Abrégé**

L'invention a pour objet de nouveaux copolymères linéaires ou réticulés constitués d'unités monomériques issues de la Tupalin A et de l'acide itaconique, le procédé pour leur préparation, leur utilisation en dermocosmétique ou en dermopharmacie et les formulations topiques en comportant.

## Description

### DOMAINE TECHNIQUE DE L'INVENTION

L'invention relève du domaine des industries cosmétique et pharmaceutique. L'invention concerne plus particulièrement de nouveaux agents épaississants d'origine naturelle.

### ÉTAT DE LA TECHNIQUE ANTÉRIEURE

Les polymères sont largement utilisés aujourd'hui dans les formulations à usage topique pour des utilisations dans les domaines de la cosmétique, de la dermocosmétique, de la pharmacie ou de la dermopharmacie. La plupart d'entre eux sont des modificateurs de rhéologie, qui épaississent les phases polaires, comme l'eau. Parmi les polymères synthétiques épaississants de phase aqueuse, on retrouve les polyélectrolytes, linéaires ou réticulés, qui ont la capacité de se déployer dans un solvant polaire, du fait de leur affinité avec ces phases et grâce aux répulsions électrostatiques dues à la présence des charges sur leur squelette polymérique. Ce déploiement dans les phases polaires, créant un réseau polymérique, se traduit par une augmentation de la viscosité et l'apport d'une consistance ainsi qu'une stabilisation dans les formules contenant des huiles, type gel-crème ou émulsion.

Comme exemples de tels polymères, il y a les polyélectrolytes réticulés, qui ont la capacité de se déployer dans le solvant polaire notamment grâce aux forces de répulsions électrostatiques dues à la présence de charges électriques sur leur squelette polymérique. Ce phénomène permet ainsi de former un réseau polymérique qui induit une augmentation de la viscosité, une consistance et une stabilisation de Formulations comme les émulsions de type huile-dans-eau ou eau-dans-huile en présence ou non de tensioactifs émulsionnants ou de gel-crèmes exempts de tensioactifs émulsionnants. Ces polymères, résultent généralement de la polymérisation de monomères de type acrylate ou méthacrylate ou encore de monomères dérivés de l'acrylamide ou de ses dérivés.

Comme polyélectrolytes réticulés commercialisés sur le marché ou divulgués dans la littérature, il y a par exemple :
L'homopolymère de l'acide acrylique partiellement ou totalement salifié par un sel de sodium, d'ammonium ou de potassium, l'homopolymère de l'acide méthacrylique partiellement ou totalement salifié par un sel de sodium, d'ammonium ou de potassium,
l'homopolymère de l'acide 2-méthyl-((1-oxo-2-propényl) amino)-1-propanesulfonique (AMPS) partiellement ou totalement salifié par un sel de sodium, d'ammonium ou de potassium,
les copolymères de l'acide acrylique, partiellement ou totalement salifié par un sel de sodium, d'ammonium ou de potassium et de l'AMPS, les copolymères de l'acide
méthacrylique, partiellement ou totalement salifié par un sel de sodium, d'ammonium ou de potassium et de l'AMPS,
les copolymères de l'acrylamide et de l'AMPS partiellement ou totalement salifié par un sel de sodium, d'ammonium ou de potassium,
les copolymères de la vinylpyrolidone et de l'AMPS partiellement ou totalement salifié par un sel de sodium, d'ammonium ou de potassium,
les copolymères de l'AMPS partiellement ou totalement salifié par un sel de sodium, d'ammonium ou de potassium et de l'acrylate de (2-hydroxy éthyle) ou du méthacrylate de (2-hydroxy éthyle), ou l'acrylate de (2,3-dihydroxy propyle) ou le méthacrylate de (2,3-dihydroxy propyle),
les copolymères de l'AMPS partiellement ou totalement salifié par un sel de sodium, d'ammonium ou de potassium et de l'hydroxyéthylacrylamide, ou de l'hydroxyéthylméthacrylamide,
les copolymères de l'AMPS partiellement ou totalement salifié par un sel de sodium, d'ammonium ou de potassium et du N,N-diméthyl acrylamide, du N,N-diméthyl méthacrylamide, du N,N-diéthyl acrylamide, du N,N-diéthyl méthacrylamide, du N-méthyl acrylamide, du N-éthyl acrylamide, du N-propyl acrylamide, du N-isopropyl acrylamide, du N-butyl acrylamide, du N-(tert-butyl) acrylamide, du N-méthyl méthacrylamide, du N-éthyl méthacrylamide, du N-propyl méthacrylamide, du N-isopropyl méthacrylamide, du N-butyl méthacrylamide, du N-(tert-butyl) méthacrylamide ou du N,N-dipropyl acrylamide,
les copolymères de l'AMPS partiellement ou totalement salifié par un sel de sodium, d'ammonium ou de potassium et du tris(hydroxyméthyl) acrylamido méthane (THAM),
les copolymères de l'acide acrylique ou de l'acide méthacrylique, partiellement ou totalement salifiés par un sel de sodium, d'ammonium ou de potassium, et de l'acrylate de (2-hydroxy éthyle), ou l'acrylate de (2,3-dihydroxy propyle),
les copolymères de l'acide acrylique ou de l'acide méthacrylique, partiellement ou totalement salifiés par un sel de sodium, d'ammonium ou de potassium, et du méthacrylate de (2-hydroxy éthyle), ou le méthacrylate de (2,3-dihydroxy propyle),
les copolymères de l'acide acrylique ou de l'acide méthacrylique partiellement ou totalement salifiés par un sel de sodium, d'ammonium ou de potassium, et de l'hydroxyéthylacrylamide,
les copolymères de l'acide acrylique ou de l'acide méthacrylique partiellement ou totalement salifiés par un sel de sodium, d'ammonium ou de potassium et du THAM,
les copolymères de l'acide acrylique ou de l'acide méthacrylique partiellement ou totalement salifiés par un sel de sodium, d'ammonium ou de potassium et du N,N-diméthyl acrylamide, du N,N-diméthyl méthacrylamide, du N,N-diéthyl acrylamide, du N,N-diéthyl méthacrylamide, du N-méthyl acrylamide, du N-éthyl acrylamide, du N-propyl acrylamide, du N-isopropyl acrylamide, du N-butyl acrylamide, du N-(tert-butyl) acrylamide, du N-méthyl méthacrylamide, du N-éthyl méthacrylamide, du N-propyl méthacrylamide, du N-isopropyl méthacrylamide, du N-butyl méthacrylamide, du N-(tert-butyl)méthacrylamide, ou du N,N-dipropyl acrylamide,
les terpolymères branchés ou réticulés de l'acide acrylique ou de l'acide méthacrylique partiellement ou totalement salifiés par un sel de sodium, d'ammonium ou de potassium, de l'AMPS partiellement ou totalement salifié par un sel de sodium, d'ammonium ou de potassium et de l'acrylate de (2-hydroxy éthyle), du méthacrylate de (2-hydroxy éthyle), de l'acrylate de (2,3-dihydroxy propyle) ou du méthacrylate de (2,3-dihydroxy propyle),
les terpolymères branchés ou réticulés de l'acide acrylique ou de l'acide méthacrylique partiellement ou totalement salifiés par un sel de sodium, d'ammonium ou de potassium, de l'AMPS partiellement ou totalement salifié par un sel de sodium, d'ammonium ou de potassium et du THAM,
les terpolymères branchés ou réticulés de l'acide acrylique ou de l'acide méthacrylique partiellement ou totalement salifiés par un sel de sodium, d'ammonium ou de potassium, de l'AMPS partiellement ou totalement salifié par un sel de sodium, d'ammonium ou de potassium et du N,N-diméthyl acrylamide, du N,N-diméthyl méthacrylamide, du N,N-diéthyl acrylamide, du N,N-diéthyl méthacrylamide, du N-méthyl acrylamide, du N-éthyl acrylamide, du N-propyl acrylamide, du N-isopropyl acrylamide, du N-butyl acrylamide, du N-(tert-butyl) acrylamide, du N-méthyl méthacrylamide, du N-éthyl méthacrylamide, du N-propyl méthacrylamide, du N-isopropyl méthacrylamide, du N-butyl méthacrylamide, du N-(tert-butyl)méthacrylamide, ou du N,N-dipropyl acrylamide,
les terpolymères branchés ou réticulés de l'acide acrylique ou de l'acide méthacrylique partiellement ou totalement salifiés par un sel de sodium, d'ammonium ou de potassium, de l'AMPS partiellement ou totalement salifié par un sel de sodium, d'ammonium ou de potassium et de l'acrylamide ou du méthacrylamide,
les copolymères de l'acide acrylique ou de l'acide méthacrylique partiellement ou totalement salifiés par un sel de sodium, d'ammonium ou de potassium, et d'acrylates d'alkyle ou de méthacrylate d'alkyle dont la chaîne carbonée comprend entre quatre et trente atomes de carbone et plus particulièrement entre dix et trente atomes de carbone, et
les copolymères de l'AMPS partiellement ou totalement salifiés par un sel de sodium, d'ammonium ou de potassium et d'acrylates d'alkyle ou de méthacrylate d'alkyle dont la chaîne carbonée comprend entre quatre et trente atomes de carbone et plus particulièrement entre dix et trente atomes de carbone.

Aujourd'hui ces polymères, qu'ils soient sous forme de latex inverses auto-inversibles, de latex inverses concentrés ou de poudres, permettent de répondre aux besoins clients en termes de performances épaississantes, dans un solvant polaire comme l'eau. Les gels aqueux obtenus par leur dispersion dans ce solvant, présentent un aspect lisse, exempts de grains ou grumeaux, avec des propriétés sensorielles particulières au toucher, ainsi qu'une facilité de préhension et d'application sur la peau.

Malgré l'offre commerciale importante, la demande de nouveaux épaississants de phases aqueuses pour la formulation de produits cosmétiques et dermopharmaceutiques reste d'actualité car les polymères utilisés aujourd'hui sont majoritairement d'origine pétrochimique, alors que les industries utilisatrices sont engagées dans une démarche d'éco-conception. Il est donc nécessaire de développer des épaississants alternatifs qui, tout en ayant des propriétés comparables, ont un profil environnemental amélioré, soit par leur origine ou celle de leurs précurseurs, soit par leur caractère biodégradable.

Il est connu de l'homme du métier que les hydrocolloïdes, polysaccharides ou protéines, peuvent présenter des propriétés épaississantes, gélifiantes et stabilisantes. Mais très souvent, ces produits naturels présentent des propriétés sensorielles non satisfaisantes, en procurant aux formules cosmétiques, un toucher collant, filant et savonnant.

Les inventeurs ont donc chercher à développer de nouveaux polymères épaississants qui soient synthétisés à partir de monomères d'origine naturelle et qui procurent aux formulations topiques cosmétiques ou pharmaceutiques un profil sensoriel accepté par l'utilisateur final.

### EXPOSE DE L'INVENTION

C'est la raison pour laquelle, selon un premier aspect l'invention a pour objet un copolymère linéaire ou réticulé constitué d'unités monomériques issues du 3-méthylène-dihydro-2(3H)-furanone de formule (I) : 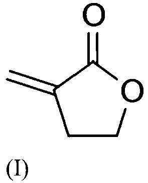
et d'unités monomériques issues de l'acide méthylène-1,4-butanedioïque de formule (II) : 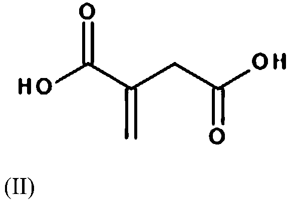
dans lequel le rapport molaire RA ayant comme numérateur le nombre de moles d'unités monomériques issues de composé de formule (I) et comme dénominateur le nombre de moles d'unités monomériques issues du composé de formule (II) est supérieur ou égal à 0,4 et inférieur ou égal à 3,0.

Dans le cadre de la présente invention les monomères de formules (I) et de formule (II) tel que définis ci-dessus sont disponibles dans le commerce et peuvent être obtenus à partir de biomasse. L'alpha-méthylène-gamma-butyrolactone est connu sous le nom de Tulipalin A et l'acide méthylène-1,4-butanedioïque de formule (II) est connu sous le numéro de d'acide itaconique.

Selon un aspect particulier de la présente invention le copolymère linéaire ou réticulé selon tel que défini précédemment est caractérisé en ce que ledit rapport molaire RA est supérieur ou égal à 1,5 et inférieur ou égal à 2,5.

Selon un autre aspect particulier de la présente invention lorsque le copolymère tel que défini précédemment est réticulé et le rapport molaire RB ayant comme numérateur le nombre de moles d'unités monomériques issues du monomère de réticulation et comme dénominateur la somme des nombres de moles d'unités monomériques issues du composé de formule (I) et de moles d'unités monomériques issues du composé de formule (II) est supérieur ou égal à 0,0010 et inférieur ou égal à 0,0070 et est plus particulièrement supérieur ou égal à 0,0020 et inférieur ou égal à 0,0060.

Selon un autre aspect particulier de la présente invention lorsque le copolymère tel que défini précédemment est réticulé, les unités monomériques issues du monomère de réticulation, le sont d'un composé choisi dans le groupe constitué par le méthylène bis(acrylamide) (MBA) (Nombre CA = 110-26-9), le tétraacrylate de pentaérythritol (PETA) (Nombre CA = 4986-89-4), le triacrylate de triméthylol propane (TMPTA) (Nombre CA = 15625-89-5), la triallylamine (TAA), le pentaérythritol triallyléther (APE) (Nombre CA = 1471-17-6), le diacrylate de 1,6-hexanediol (HDDA) (Nombre CA = 13048-33-4), le diacrylate d'éthylèneglycol (EGDA) (Nombre CA = 2274-11-5) et le diallyloxyacétate de sodium (DAOAS) et, plus particulièrement dans le groupe constitué par le tétraacrylate de pentaérythritol (PETA) et le triacrylate de triméthylol propane (TMPTA).

L'invention a aussi pour objet un procédé de préparation du copolymère linéaire ou réticulé tel que défini ci-dessus comprenant les étapes successives suivantes :
une étape a) de mise à disposition :
d'un solvant choisi dans le groupe comprenant l'eau, l'éthanol, le propanol, l'isopropanol, le butanol, l'isobutanol, les sec-butanol, tert-butanol, l'acétone, la méthyl éthyl cétone, le cyclohexane, l'acétate d'éthyle ou un mélange de deux ou de plusieurs d'entre eux ;
dudit monomère de formule (II) et dudit monomère de formule (I) dans des proportions molaires telles que le rapport molaire R'A ayant comme numérateur le nombre de moles de composé de formule (1) et comme dénominateur le nombre de moles de composé de formule (II) soit supérieur ou égal à 0,4 et inférieur ou égal à 3,0 ; et
optionnellement dudit monomère de réticulation choisi dans le groupe constitué par le méthylène bis(acrylamide), le tétraacrylate de pentaérythritol, le triacrylate de triméthylol propane, la triallylamine pentaérythritol triallyléther, le diacrylate de 1,6-hexanediol, le diacrylate d'éthylèneglycol et le diallyloxyacétate de sodium, dans des proportions molaires telles que le rapport molaire R'B ayant comme numérateur le nombre de moles de monomère de réticulation et comme dénominateur la somme des nombres de moles de composé de formule (I) et de composé de formule (II), soit supérieur ou égal à 0,001 et inférieur ou égal à 0,007 ;
une étape b) d'addition de la totalité des moles de composé de formule (II) dans le solvant mis à disposition à l'étape a) pour former une solution ou une dispersion dudit composé de formule (II),
optionnellement une étape c) de neutralisation partielle ou totale du composé de formula (II), en ajoutant une base dans ladite solution ou dispersion formée à l'étape b), pour former une solution ou une dispersion dudit composé de formule (II) partiellement ou totalement neutralisé ;
une étape d) d'addition soit d'une partie, soit de la totalité des moles de composé de formule (I) mises à disposition à l'étape a) dans ladite solution ou dispersion formée à l'étape b) ou dans ladite solution ou dispersion formée à l'étape c), pour former une solution ou une dispersion dudit composé de formule (II), optionnellement partiellement ou complètement neutralisé, et dudit composé de formule (I) ;
optionnellement une étape e) d'addition soit d'une partie, soit de la totalité des moles de monomère de réticulation dans ladite solution ou dispersion obtenue à l'étape d), pour former une solution ou une dispersion dudit composé de formule (II), optionnellement partiellement ou complètement neutralisé, dudit composé de formule (I) et optionnellement dudit monomère de réticulation ;
une étape f) de désoxygénation de ladite solution ou dispersion obtenue à l'étape d), respectivement à l'étape optionnelle e), par barbotage en son sein d'un gaz inerte, plus particulièrement d'azote, pendant une durée supérieure ou égale 30 minutes et inférieure ou égale à 90 minutes, pour obtenir une solution ou une dispersion désoxygénée dudit composé de formule (II), optionnellement partiellement ou complètement neutralisé, dudit composé de formule (I) et optionnellement dudit monomère de réticulation ;
une étape g) de chauffage à une température supérieure ou égale à 50°C et inférieure ou égale à 95°C de ladite solution ou dispersion obtenue à l'étape f), pour obtenir une solution ou une dispersion désoxygénée et chauffée dudit composé de formule (II), optionnellement partiellement ou complètement neutralisé, dudit composé de formule (I) et optionnellement dudit monomère de réticulation ;
une étape h) de polymérisation des monomères présents au sein de ladite solution ou dispersion désoxygénée et chauffée obtenue à l'étape g), par addition d'un amorceur de polymérisation, plus particulièrement le peroxyde dilauroyle ou le dichlorhydrate 2,2'-azobis(2-méthylpropionamidine) et maintien à une température de polymérisation supérieure ou égale à 50°C et inférieure ou égale à 95°C, pendant une durée supérieure ou égale à 150 minutes et inférieure ou égale à 300 minutes, pour obtenir un précipité dudit copolymère optionnellement réticulé attendu et optionnellement dudit monomère de formule (II) n'ayant pas réagi au sein dudit solvant mis à disposition à l'étape a) ;
optionnellement une étape i) d'addition dans ledit solvant obtenu à l'étape h), comprenant le précipité dudit copolymère optionnellement réticulé attendu et optionnellement ledit monomère de formule (II) n'ayant pas réagi, du reste dudit composé de formule (I) et optionnellement du reste dudit monomère de réticulation mis à disposition à l'étape a), puis de polymérisation du reste des monomères présents par addition d'un amorceur de polymérisation, particulièrement le peroxyde dilauroyle ou le dichlorhydrate de 2,2'-azobis(2-méthylpropionamidine) en maintenant la température à une valeur supérieure ou égale à 50°C et inférieure ou égale à 95°C, pendant une durée supérieure ou égale à 150 minutes et inférieure ou égale à 300 minutes, pour obtenir une dispersion dudit copolymère optionnellement réticulé attendu ;
une étape j) de refroidissement de ladite dispersion dudit copolymère optionnellement réticulé obtenu à l'étape i) jusqu'à une température supérieure ou égale à 15°C et inférieure ou égale à 30°C; et
soit une étape k) de filtration de ladite dispersion refroidie obtenue à l'étape j), pour recueillir ledit copolymère optionnellement réticulé attendu suivie d'une étape I) de séchage dudit copolymère optionnellement réticulé recueilli,
soit une étape m) d'atomisation ladite dispersion refroidie obtenue à l'étape j), pour obtenir ledit copolymère optionnellement réticulé attendu sous forme de poudre.

Selon un aspect particulier du procédé de préparation du copolymère linéaire ou réticulé tel que défini ci-dessus, les étapes b), c) optionnelle, d) et e) optionnelle sont simultanées et constituent une étape B).

Selon un autre aspect particulier du procédé de préparation du copolymère linéaire ou réticulé tel que défini ci-dessus, le solvant mis à disposition à l'étape a) est soit l'eau, soit le tert-butanol soit un mélange tert-butanol-eau.

Selon cet aspect particulier ledit procédé du copolymère linéaire ou réticulé comprend plus particulièrement les étapes successives suivantes :
une étape a) de mise à disposition :
   d'un mélange tert-butanol-eau comprenant pour 100% massique, une proportion massique supérieure ou égale à 90% et inférieure ou égale à 98% massique de tert-butanol et une proportion massique supérieure ou égale à 2% massique et inférieure ou égale à 10% massique d'eau ;
   dudit monomère de formule (II) et dudit monomère de formule (I) dans des proportions molaires telles que le rapport molaire R'A ayant comme numérateur le nombre de moles de composé de formule (I) et comme dénominateur le nombre de moles de composé de formule (II) soit supérieur ou égal à 1,0 et inférieur ou égal à 3,0 ; et optionnellement ;
   dudit monomère de réticulation choisi dans le groupe constitué par le le tétraacrylate de pentaérythritol et le triacrylate de triméthylol propane, dans des proportions molaires telles que le rapport molaire R'B ayant comme numérateur le nombre de moles de monomère de réticulation et comme dénominateur la somme des nombres de moles de composé de formule (I) et de composé de formule (II), soit supérieur ou égal à 0,003 et inférieur ou égal à 0,005 ;
une étape B) d'addition dans ledit mélange tert-butanol-eau dans le solvant mis à disposition à l'étape a) de la totalité des moles de composé de formule (II) dudit composé de formule (II), de 50% des moles de composé de formule (I) et optionnellement de 50% des moles dudit monomère de réticulation pour former une solution ou une dispersion dudit composé de formule (II), dudit composé de formule (I) et optionnellement dudit monomère de réticulation ;
une étape f) de désoxygénation de ladite solution ou dispersion obtenue à l'étape B), par barbotage d'azote en son sein, pendant une durée supérieure ou égale 45 minutes et inférieure ou égale à 60 minutes, pour obtenir une solution ou une dispersion désoxygénée dudit composé de formule (II), dudit composé de formule (I) et optionnellement dudit monomère de réticulation ;
une étape g) de chauffage à une température supérieure ou égale à 50°C et inférieure ou égale à 85°C de ladite solution ou dispersion obtenue à l'étape f), pour obtenir une solution ou une dispersion désoxygénée et chauffée dudit composé de formule (II), dudit composé de formule (I) et optionnellement dudit monomère de réticulation;
une étape h) de polymérisation des monomères présents au sein de ladite solution ou dispersion désoxygénée et chauffée obtenue à l'étape g), par addition de peroxyde dilauroyle et maintien à une température de polymérisation supérieure ou égale à 75°C et inférieure ou égale à 85°C, pendant un durée supérieure ou égale à 150 minutes et inférieure ou égale à 200 minutes pour obtenir une précipité dudit copolymère optionnellement réticulé attendu et optionnellement dudit monomère de formule (II) n'ayant pas réagi au sein dudit solvant mis à disposition à l'étape a) et ;
une étape i) d'addition dans ledit solvant obtenu à l'étape h), comprenant le précipité dudit copolymère optionnellement réticulé attendu et optionnellement ledit monomère de formule (II) n'ayant pas réagi, du reste dudit composé de formule (I) et optionnellement du reste dudit monomère de réticulation mis à disposition à l'étape a), puis de polymérisation du reste des monomères présents par addition de peroxyde dilauroyle et maintien à une température de polymérisation supérieure ou égale à 75°C et inférieure ou égale à 85°C, pendant une durée supérieure ou égale à 150 minutes et inférieure ou égale à 200 minutes, pour obtenir une dispersion dudit copolymère optionnellement réticulé attendu ;
une étape j) de refroidissement de ladite dispersion dudit copolymère optionnellement réticulé obtenu à l'étape i) jusqu'à une température supérieure ou égale à 15°C et inférieure ou égale à 30°C; et
soit une étape k) de filtration de ladite dispersion refroidie obtenue à l'étape j), pour recueillir ledit copolymère optionnellement réticulé attendu suivie d'une étape I) de séchage dudit copolymère optionnellement réticulé recueilli,
soit une étape m) d'atomisation ladite dispersion refroidie obtenue à l'étape j) pour obtenir ledit copolymère optionnellement réticulé attendu sous forme de poudre.

Le procédé tel que défini ci-dessus permet d'obtenir des copolymères d'origine naturelle qui sont des substituts intéressants au polymères d'origine pétrolière encore utilisés aujourd'hui en dermocosmétique ou en dermopharmacie.

C'est pourquoi l'invention a aussi pour objet l'utilisation du copolymère linéaire ou réticulé tel que défini précédemment, en vue d'épaissir, de stabiliser ou d'émulsionner une formulation topique cosmétique ou pharmaceutique ou d'y suspendre des particules solides ; ainsi qu'une formulation topique cosmétique ou pharmaceutique caractérisée en ce qu'elle comprend pour 100,0% de sa masse, de 0,1% à 10,0% massique et plus particulièrement de 0,5% à 5,0% massique du copolymère linéaire ou réticulé tel que défini précédemment, comme agent épaississant, comme agent stabilisant ou comme agent émulsionnant de ladite formulation topique cosmétique ou pharmaceutique ou comme agent apte et destiné à suspendre des particules solides au sein de ladite formulation topique cosmétique ou pharmaceutique.

Les particules solides suspendues dans la formulations topique cosmétique ou pharmaceutique objet de l'utilisation ou de l'invention telle que définie ci-dessus, ont une géométrie régulière ou irrégulière, et se présentent généralement sous forme de perles, de billes, de tiges, de paillettes, de lamelles ou de polyèdres. Elles se caractérisent par un diamètre moyen apparent compris entre un micromètre et cinq millimètres, plus particulièrement entre dix micromètres et un millimètre. Comme exemples de telles particules solides, il y a il y a les micas, l'oxyde de fer, l'oxyde de titane, l'oxyde de zinc, l'oxyde d'aluminium, le talc, la silice, le kaolin, les argiles, le nitrure de bore, le carbonate de calcium, le carbonate de magnésium, l'hydrogénocarbonate de magnésium, les pigments colorés inorganiques, les polyamides comme le nylon-6, les polyéthylènes, les polypropylènes, les polystyrènes, les polyesters, les polymères acryliques ou méthacryliques comme les polyméthylméthacrylates, le polytétrafluoroéthylène, les cires cristallines ou microcristallines, des sphères poreuses, le sulfure de sélénium, le pyrithione de zinc, les amidons, les alginates, les fibres de végétaux, les particules de Loofah ou les particules d'éponges.

Dans la formulation topique cosmétique ou pharmaceutique objet de l'utilisation ou de l'invention telle que définie ci-dessus, l'adjectif topique signifie que ladite formulation est mise en œuvre par application sur la peau, les cheveux, le cuir chevelu ou les muqueuses, qu'il s'agisse d'une application directe ou d'une application indirecte lorsque la composition topique selon l'invention est imprégnée sur un support destine à être mis en contact avec la peau (papier, lingette, textile, dispositif transdermique, etc.).

La formulation topique cosmétique ou pharmaceutique objet de l'utilisation ou de l'invention telle que définie ci-dessus, peut se présenter sous toute forme physique, par exemple sous la forme d'un gel aqueux hydro-alcoolique ou hydro-glycolique; d'une solution; d'une poudre; d'une suspension, d'une émulsion, d'une microémulsion ou d'une nano-émulsion, qu'elles soient de type eau-dans-huile, huile-dans-eau, eau-dans-huile-dans-eau ou huile-dans-eau-dans-huile. Elle peut être conditionnée dans un flacon, dans un dispositif de type "flacon" pompe, sous forme pressurisée dans un dispositif aérosol, dans un dispositif muni d'une paroi ajourée comme une grille ou dans un dispositif muni d'un applicateur à bille (dit "roll-on").

De façon générale, la formulation topique cosmétique ou pharmaceutique objet de l'utilisation ou de l'invention telle que définie ci-dessus, peut également comprendre un ou plusieurs composants ingrédients cosmétiquement ou pharmaceutiquement acceptables, qu'il s'agisse de principes actifs ou d'excipients comme les tensioactifs moussants, non moussants ou détergents, les agents épaississants ou gélifiants, les agents stabilisants, les agents solubilisants, les composés filmogènes, les solvants et co-solvants, les agents hydrotropes, les eaux thermales ou minérales les agents plastifiants, les agents émulsionnants et co-émulsionnants, les agents opacifiants, les agents nacrant, les agents surgraissants, les séquestrants, les agents chélatants, les huiles, les cires, les agents antioxydants, les parfums, les huiles essentielles, les agents conservateurs, les agents conditionneurs, les agents déodorants, les agents blanchissants destines à la décoloration des poils et de la peau, les principes actifs destinés à apporter une action traitante ou protectrice vis à vis de la peau ou des cheveux, les filtres solaires, les charges minérales ou les pigments, les particules procurant un effet visuel ou destinées à l'encapsulation d'actifs, les particules exfoliantes, les agents de texture, les azurants optiques, les répulsifs pour les insectes.

L'expression « cosmétiquement acceptable » utilisée dans la définition des ingrédients susmentionnés, signifie selon la directive du Conseil de la Communauté Economique Européenne N° 76/768/CEE du 27 juillet 1976 modifiée par la directive N°93/35/CEE du 14 juin 1993, que ledit excipient (E) comprend de l'eau et toute substance ou préparation destinée à être mise en contact avec les diverses parties du corps humain (épiderme, système pileux et capillaire, ongles, lèvres et organes génitaux) ou avec les dents et les muqueuses buccales en vue, exclusivement et principalement, de les nettoyer, de les parfumer, d'en modifier l'aspect ou d'en corriger les odeurs corporelles ou de les protéger ou de les maintenir en bon état.

L'expression « pharmaceutiquement acceptable » utilisée dans la définition des ingrédients susmentionnés, signifie que ces ingrédients sont inscrits dans la Pharmacopée de l'Etat dans lequel ladite formulation est utilisée.

Comme exemples de tensioactifs moussants, optionnellement présents dans la formulation topique cosmétique ou pharmaceutique objet de l'utilisation ou de l'invention telles que définies ci-dessus, il y a les tensioactifs moussants ou détergents anioniques, cationiques, amphotères ou non ioniques.

Parmi les tensioactifs anioniques moussants, on peut citer les sels de métaux alcalins, les sels de métaux alcalino-terreux, les sels d'ammonium, les sels d'amines ou les sels d'amino-alcools :
des alkyl éthers sulfates, comme le C12-14 myristyl éther sulfate de sodium à 3,5 moles d'oxyde d'éthylène, le C12-14 lauryl éther sulfate de sodium à 2 moles d'oxyde d'éthylène, le C12-14 lauryl éther sulfate d'ammonium à 2 moles d'oxyde d'éthylène, le C12-14 lauryl éther sulfate de mono isopropyl amine à 2 moles d'oxyde d'éthylène dans le propylène glycol, le C12-14 lauryl éther sulfate de tri isopropyl amine à 2 moles d'oxyde d'éthylène dans le propylène glycol, le C12-14 lauryl éther sulfate de sodium à 3 moles d'oxyde d'éthylène, le C12-14 lauryl éther sulfate d'ammonium à 3 moles d'oxyde d'éthylène, le C12-15 lauryl éther sulfate de sodium à 3 moles d'oxyde d'éthylène, le C8-10 lauryl éther sulfate de sodium à 3 moles d'oxyde d'éthylène, le C8-10 lauryl éther sulfate d'ammonium à 3 moles d'oxyde d'éthylène, le C9-11 lauryl éther sulfate de sodium à 2,5 moles d'oxyde d'éthylène, le C9-11 lauryl éther sulfate d'ammonium à 2,5 moles d'oxyde d'éthylène, le C9-11 lauryl éther sulfate d'ammonium à 2,5 moles d'oxyde d'éthylène dans l'hexylène glycol ;
des alkylsulfates comme le lauryl sulfate de sodium, le lauryl sulfate de potassium, le lauryl sulfate d'ammonium, le lauryl sulfate de magnésium, le cocoyl sulfate de sodium, le cocoyl sulfate de potassium, le cocoyl sulfate d'ammonium, le cocoyl sulfate de magnésium ;
des alkylamidoéthersulfates,des alkylarylpolyéthersulfates, des monoglycérides sulfates, des alpha-oléfinesulfonates, des paraffines sulfonates ;
des alkylphosphates, des alkylétherphosphates, des alkylsulfonates, des alkylamides sulfonates, des alkylarylsulfonates, des alkylcarboxylates, des alkylsulfosuccinates, des alkyléthersulfosuccinates, des alkylamidesulfosuccinates, des alkylsulfoacétates ;
des dérivés N-acylés d'acides aminés, comme le lauroyl sarcosinate de sodium, le lauroyl sarcosinate de potassium, le lauroyl sarcosinate de magnésium, le lauroyl sarcosinate d'ammonium, le lauroyl glycinate de sodium, le lauroyl glycinate de potassium, le lauroyl glycinate de magnésium, le lauroyl glycinate d'ammonium, le cocoyl sarcosinate de sodium, le cocoyl sarcosinate de potassium, le cocoyl sarcosinate de magnésium, le cocoyl sarcosinate d'ammonium, le cocoyl glycinate de sodium, le cocoyl glycinate de potassium, le cocoyl glycinate de magnésium, le cocoyl glycinate d'ammonium, le cocoyl glutamate de sodium, le cocoyl glutamate de potassium, le cocoyl glutamate de magnésium, le cocoyl glycinate d'ammonium, le cocoyl aspartate de sodium, le cocoyl aspartate de potassium, le cocoyl aspartate de magnésium, le cocoyl aspartate d'ammonium ; ou des mélanges comprenant lesdits dérivés N-acylés d'acides aminés comme ceux par exemple commercialisés sous le nom de marque Proteol^{™}OAT, Proteol^{™}APL, Oramix^{™}L30 ;
des acyliséthionates, comme le cocoyl iséthionate de sodium, le cocoyl iséthionate de potassium, le cocoyl iséthionate de magnésium, le cocoyl iséthionate d'ammonium, le lauroyl iséthionate de sodium, le lauroyl iséthionate de potassium, le lauroyl iséthionate de magnésium, le lauroyl iséthionate d'ammonium ;
des N-acyltaurates, comme le méthyl cocoyl taurate de sodium, le méthyl cocoyl taurate de potassium, le méthyl cocoyl taurate de magnésium, ,le méthyl cocoyl taurate d'ammonium ou
des acyllactylates.

Parmi les tensioactifs amphotères moussants, on peut citer :
les alkylbétaïnes, comme la lauryl bétaïne, la cocoyl bétaïne, la myristyl bétaïne ;
les alkylamidobétaïnes, comme la lauramidopropyl bétaïne, le sel de sodium de la cocamidopropyl bétaïne, la 1-propanaminium, 3-amino-N-(carboxyméthyl)-N,N-diméthyl-, ou la composition commercialisée sous le nom de marque Amonyl^{™}380 BA;
les sultaïnes, les alkylamidoalkylsulfobétaïnes, ou la composition commercialisée sous le nom de marque Amonyl^{™}675 SB ou
les dérivés d'imidazolines, les phosphobétaïnes, les amphopolyacétates et les amphopropionates.

Parmi les tensioactifs cationiques moussants, on peut citer particulièrement les dérivés d'ammoniums quaternaires.

Parmi les tensioactifs non ioniques moussants, on peut citer plus particulièrement les alkylpolyglycosides comportant un radical aliphatique, linéaire ou ramifié, saturé ou insaturé, et comportant de 8 à 16 atomes de carbone, comme le n-octyl polyglucoside, le n-décyl polyglucoside, n-undécylényl polyglucoside, le n-dodécyl polyglucoside, le n-tétradécyl polyglucoside, le n-hexadécyl polyglucoside, le 1-12 dodécanediyl polyglucoside ; les dérivés d'huile de ricin hydrogénée éthoxylés comme le produit commercialisé sous le nom INCI « Peg-40 hydrogenated castor oil » ; les polysorbates comme le Polysorbate 20, le Polysorbate 40, le Polysorbate 60, le Polysorbate 70, le Polysorbate 80, le Polysorbate 85 ; les amides de coprah ; les N-alkylamines ;

Comme exemples d'agents épaississants ou gélifiants optionnellement présents dans la formulation topique cosmétique ou pharmaceutique objet de l'utilisation ou de l'invention telles que définies ci-dessus, on peut citer :
les esters gras d'alkylpolyglycosides éventuellement alcoxylés, comme les esters de méthylpolyglucoside éthoxylés tels que le PEG 120 méthyl glucose trioléate et le PEG 120 méthyl glucose dioléate commercialisés respectivement sous les appellations GLUCAMATE^{™} LT et GLUMATE^{™} DOE120 ;
les esters gras alcoxylés tels que le PEG 150 pentaérythrytyl tétrastéarate commercialisé sous l'appellation CROTHIX^{™} DS53,
le PEG 55 propylène glycol oléate commercialisé sous l'appellation ANTIL^{™} 141,
les carbamates de polyalkylène glycols à chaînes grasses comme le PPG-14 laureth isophoryl dicarbamate commercialisé sous l'appellation ELFACOS^{™} T211, ou le PPG-14 palmeth-60 hexyl dicarbamate commercialisé sous l'appellation ELFACOS^{™} GT2125 ;
la cellulose, les dérivés de la cellulose comme la méthyl-cellulose, l'éthyl-cellulose, l'hydroxy-éthyl cellulose, l'hydroxy-propyl cellulose, l'hydroxy-propyl méthyl cellulose, la carboxy méthyl cellulose,
l'amidon, les dérivés hydrophiles de l'amidon,
les polyuréthanes,
les silicates et les phyllosilicates comme les silicates d'aluminium, les silicates de magnésium, aluminium ou de magnésium, la kaolinite, la montmorillonite, l'illite, la beidellite, la saponite, la bentonite, l'hectorite, la vermiculite, la serpentine, la nacrite, l'amésite, la nontronite, la lizardite, la beidellite, la séricite, l'halloylsite, la muscovite, la paragonite, la damouzite, la glauconite ou la céladonite ;
les polyélectrolytes branchés ou réticulés, anioniques, cationiques ou amphotères comme les homopolymères branchés ou réticulés de l'acide acrylique, de l'acide méthacrylique ou de l'acide 2-méthyl-[(1-oxo-2-propényl)amino]-1-propanesulfonique (AMPS) partiellement ou totalement salifiés par un sel de sodium, d'ammonium ou de potassium ; les copolymères de l'acide acrylique ou de l'acide méthacrylique, partiellement ou totalement salifiés par un sel de sodium, d'ammonium ou de potassium et de l'AMPS partiellement ou totalement salifié par un sel de sodium, d'ammonium ou de potassium ; les copolymères branchés ou réticulés de l'acrylamide ou de la vinylpyrolidone et de l'AMPS partiellement ou totalement salifié par un sel de sodium, d'ammonium ou de potassium ; les copolymères branchés ou réticulés de l'AMPS partiellement ou totalement salifié par un sel de sodium, d'ammonium ou de potassium et de l'acrylate de (2-hydroxyéthyle), du méthacrylate de (2-hydroxyéthyle), de l'acrylate de (2,3-dihydroxy propyle) ou du méthacrylate de (2,3-dihydroxy propyle), de l'hydroxyéthylacrylamide, de l'hydroxyéthylméthacrylamide, du N,N-diméthyl acrylamide, du N,N-diméthyl méthacrylamide, du N,N-diéthyl acrylamide, du N,N-diéthyl méthacrylamide, du N-méthyl acrylamide, du N-éthyl acrylamide, du N-propyl acrylamide, du N-isopropyl acrylamide, du N-butyl acrylamide, du N-(tert-butyl) acrylamide, du N-méthyl méthacrylamide, du N-éthyl méthacrylamide, du N-propyl méthacrylamide, du N-isopropyl méthacrylamide, du N-butyl méthacrylamide, du N-(tert-butyl)méthacrylamide, du N,N-dipropyl acrylamide ou du tris(hydroxyméthyl)acrylamido méthane (THAM) ; les copolymères branchés ou réticulés de l'acide acrylique ou de l'acide méthacrylique, partiellement ou totalement salifiés par un sel de sodium, d'ammonium ou de potassium et de l'acrylate de (2-hydroxyéthyle), du méthacrylate de (2-hydroxyéthyle), de l'acrylate de (2,3-dihydroxy propyle), du méthacrylate de (2,3-dihydroxy propyle), de l'hydroxyéthylacrylamide, de l'hydroxyéthylméthacrylamide, du N,N-diméthyl acrylamide, du N,N-diméthyl méthacrylamide, du N,N-diéthyl acrylamide, du N,N-diéthyl méthacrylamide, du N-méthyl acrylamide, du N-éthyl acrylamide, du N-propyl acrylamide, du N-isopropyl acrylamide, du N-butyl acrylamide, du N-(tert-butyl) acrylamide, du N-méthyl méthacrylamide, du N-éthyl méthacrylamide, du N-propyl méthacrylamide, du N-isopropyl méthacrylamide, du N-butyl méthacrylamide, du N-(tert-butyl)méthacrylamide, du N,N-dipropyl acrylamide ou du tris(hydroxyméthyl)acrylamido méthane (THAM) ; les terpolymères branchés ou réticulés de l'acide acrylique ou de l'acide méthacrylique partiellement ou totalement salifiés par un sel de sodium, d'ammonium ou de potassium, de l'AMPS partiellement ou totalement salifié par un sel de sodium, d'ammonium ou de potassium et de l'acrylate de (2-hydroxy éthyle), du méthacrylate de (2-hydroxy éthyle), de l'acrylate de (2,3-dihydroxy propyle) du méthacrylate de (2,3-dihydroxy propyle), de l'hydroxyéthylacrylamide, de l'hydroxyéthylméthacrylamide, du N,N-diméthyl acrylamide, du N,N-diméthyl méthacrylamide, du N,N-diéthyl acrylamide, du N,N-diéthyl méthacrylamide, du N-méthyl acrylamide, du N-éthyl acrylamide, du N-propyl acrylamide, du N-isopropyl acrylamide, du N-butyl acrylamide, du N-(tert-butyl) acrylamide, du N-méthyl méthacrylamide, du N-éthyl méthacrylamide, du N-propyl méthacrylamide, du N-isopropyl méthacrylamide, du N-butyl méthacrylamide, du N-(tert-butyl)méthacrylamide, du N,N-dipropyl acrylamide du tris(hydroxyméthyl)acrylamido méthane (THAM) ; de l'acrylamide, ou du méthacrylamide ; les copolymères branchés ou réticulés de l'acide acrylique, de l'acide méthacrylique ou de l'AMPS partiellement ou totalement salifiés par un sel de sodium, d'ammonium ou de potassium et d'un acrylate d'alkyle ou d'un méthacrylate d'alkyle dont la chaîne carbonée comprend entre quatre et trente atomes de carbone et plus particulièrement entre dix et trente atomes de carbone ; les terpolymères branchés ou réticulés de l'AMPS partiellement ou totalement salifiés par un sel de sodium, d'ammonium ou de potassium, avec au moins un monomère neutre, et au moins un monomère de formule CH₂=C(R'₃)-C(=O)-[CH₂-CH₂-O]ₙ-R' dans laquelle R'₃ représente un atome d'hydrogène ou un radical méthyle, R'₄ représente un radical alkyle linéaire ou ramifié saturé ou insaturé comportant de huit à trente atomes de carbone et plus particulièrement un radical choisi parmi les éléments du groupe constitué par le radical octyle, décyle, undécyle, dodécyle, tridécyle, tétradécyle, pentadécyle, hexadécyle, heptadécyle, octadécyle, nonadécyle, eicosyle, 2-éthyl hexyle, 2-propyl heptyle, 2-butyl octyle, 2-pentyl nonyle, 2-hexyl décyle, 2-octyl dodécyle, 4-méthyl pentyle, 5-méthyl hexyle, 6-méthyl heptyle, 15-méthyl pendadécyle, 16-méthyl heptadécyle, 2-hexyl octyle, 2-octyl décyle ou 2-hexyl dodécyle, et n représente un nombre supérieur ou égal à un et inférieur ou égal à cinquante ; les homopolymères du N,N,N-triméthyl 3-[(2-méthyl 1-oxo 2-propènyl) amino] propanammonium, du N,N,N-triméthyl 3-[(1-oxo 2-propènyl) amino] propanammonium, du diallyl diméthylammonium, du N,N,N-triméthyl 2-[(2-méthyl 1-oxo 2-propènyl)] éthanammonium, et plus particulièrement du chlorure de N,N,N-triméthyl 3-[(2-méthyl 1-oxo 2-propènyl) amino] propanammonium (MAMPTAC^{™}), du chlorure de N,N,N-triméthyl 3-[(1-oxo 2-propènyl) amino] propanammonium (APTAC^{™}) , du chlorure de diallyl diméthylammonium (DADMAC^{™}), ou du N,N,N-triméthyl 2-[(2-méthyl 1-oxo 2-propènyl)] éthanammonium (MADQUAT^{™} ; Les copolymères branchés ou réticulés du chlorure de N,N,N-triméthyl 3-[(2-méthyl 1-oxo 2-propènyl) amino] propanammonium, du chlorure de N,N,N-triméthyl 3-[(1-oxo 2-propènyl) amino] propanammonium, du chlorure de diallyl diméthylammonium ou du N,N,N-triméthyl 2-[(2-méthyl 1-oxo 2-propènyl)] éthanammonium avec l'acrylamide, le méthacrylamide, la vinylpyrolidone, l'acrylate de (2-hydroxyéthyle) le méthacrylate de (2-hydroxyéthyle), l'acrylate de (2,3-dihydroxy propyle), le méthacrylate de (2,3-dihydroxy propyle), l'hydroxyéthylacrylamide, le N,N-diméthyl acrylamide, le N,N-diméthyl méthacrylamide, le N,N-diéthyl acrylamide, le N,N-diéthyl méthacrylamide, le N-méthyl acrylamide, le N-éthyl acrylamide, le N-propyl acrylamide, le N-isopropyl acrylamide, le N-butyl acrylamide, le N-(tert-butyl) acrylamide, le N-méthyl méthacrylamide, le N-éthyl méthacrylamide, le N-propyl méthacrylamide, le N-isopropyl méthacrylamide, le N-butyl méthacrylamide, le N-(tert-butyl)méthacrylamide, le N,N-dipropyl acrylamide ou le tris(hydroxyméthyl) acrylamido méthane (THAM) ;
les polymères commercialisés sous les noms de marque CARBOPOL^{™}, PEMULEN^{™}, ARISTOFLEX^{™} , ARISTOFLEX ^{™}AVC, ARISTOFLEX ^{™}AVS, ARISTOFLEX ^{™}HMB, SEPlMAX^{™} Zen, SEPIMAX^{™} C, SEPIGEL^{™} 305, SEPIGEL^{™} 501, SEPIGEL^{™} 502, SIMULGEL^{™} 600, SIMULGEL^{™} EG, SIMULGEL^{™} EPG, SIMULGEL^{™} NS, SIMULGEL^{™} INS 100, SIMULGEL^{™} FL, SIMULGEL^{™} SMS 88, SIMULGEL^{™} 800, SIMULGEL^{™} A, SEPIPLUS^{™} 400, SEPIPLUS^{™} 250, SEPIPLUS^{™} S, SEPIPLUS^{™} NUDE, SEPILIFE^{™} G305, FIOCARE^{™} ET 25, FIOCARE^{™} ET 75, FIOCARE^{™} ET 26, FIOCARE^{™} ET 30, FIOCARE^{™} ET 58, FIOCARE^{™} PSD 30, VISCOLAM^{™} AT 64, VISCOLAM^{™} AT 100P, VISCOLAM^{™} AT EF, NOVEMER^{™} EC-1, NOVEMER^{™} EC-2, COSMEDIA^{™} SP, COSMEDIA^{™} ACE, SEPINOV^{™} EMT 10, SEPINOV^{™} WEO et SEPINOV^{™} P88;
les agents épaississants ou gélifiants de type polysaccharidique non réticulés comme les glucanes ou homopolymères du glucose, les glucomannoglucanes, les xyloglycanes, les galactomannanes dont le degré de substitution (DS) des unités de D-galactose sur la chaîne principale de D-mannose est compris entre 0 et 1, et plus particulièrement entre 1 et 0,25, comme les galactomannanes provenant de la gomme de cassia (DS = 1/5), de la gomme de caroube (DS = 1/4), de la gomme de tara (DS = 1/3), de la gomme de guar (DS = 1/2), de la gomme de fenugrec (DS = 1 ) ; les galactanes sulfatés et plus particulièrement les carraghénanes et l'agar, les uronanes et plus particulièrement les algines, les alginates et les pectines, les hétéropolymères d'oses et d'acides uroniques et plus particulièrement la gomme xanthane, la gomme gellane, les exsudats de gomme arabique et de gomme de karaya, les glucosaminoglycanes.

Les agents solubilisants mis en œuvre dans le cadre de la présente invention, sont des substances ou des compositions chimiques qui permettent de solubiliser des substances hydrophobes, insolubles dans l'eau la phase aqueuse ou dans la phase hydro-alcoolique ou dans la phase hydro-glycolique, comme les substances parfumantes et aromatisantes. Comme exemples de tels agents optionnellement présents dans la formulation topique cosmétique ou pharmaceutique objet de l'utilisation ou de l'invention telles que définies ci-dessus, il y a :
les Polysorbates, comme le Polysorbate 20, le Polysorbate 60 et le Polysorbate 80 ;
les compositions d'alkylpolyglycosides dont la chaîne alkyle linéaire ou ramifiée comprend de 4 à 10 atomes de carbone, comme le n-butylpolyglucoside, le n-butylpolyxyloside, le n-pentylpolyglucoside, le n-pentylpolyxyloside, le n-hexylpolyglucoside, le n-hexylpolyxyloside, le n-heptylpolyglucoside, le n-heptylpolyxyloside, le n-octylpolyglucoside, le n-octylpolyxyloside, le n-nonylpolyglucoside, le n-nonylpolyxyloside, le n-décylpolyglucoside, le n-décylpolyxyloside ;
les alcools gras éthoxylés de formule R10-(OE)n'-H, R10, représentant un radical aliphatique linéaire ou ramifié, saturé ou insaturé, comportant de 12 à 22 atomes de carbone, de préférence de 12 à 18 atomes de carbone, et encore particulièrement de 12 à 16 atomes de carbone, et n'représentant un nombre entier compris supérieur ou égal à 5 et inférieur ou égal à 200, plus particulièrement supérieur ou égal à 5 et inférieur ou égal à 100, plus particulièrement supérieur ou égal à 10 et inférieur ou égal à 100 ; par exemple les composés de formule R10-(OE)n'-H, R10, représentant le radical dodécyle et n représentant un nombre entier supérieur ou égal à 7 et inférieur ou égal à 25 ;
les acides gras polyéthoxylés de formule R20-C(=O)-(OE)m', R20 représentant un radical aliphatique linéaire ou ramifié, saturé ou insaturé, comportant de 12 à 22 atomes de carbone, plus particulièrement de 12 à 18 et m' représentant un nombre entier supérieur ou égal à 10 et inférieur ou égal à 100, plus particulièrement supérieur ou égal à 15 et inférieur ou égal à 100 , et encore plus particulièrement supérieur ou égal à 15 et inférieur ou égal à 50 ;
des huiles hydrogénées et éthoxylées , et plus particulièrement celles comprenant au moins un triglycéride ou un diglycéride ou un monoglycéride comme l'huile de ricin hydrogénée et éthoxylée à 40 moles d'oxyde d'éthylène commercialisée sous l'appellation « PEG-40 hydrogenated castor oil » ;

Comme exemples d'agents émulsionnants optionnellement présents dans la formulation topique cosmétique ou pharmaceutique objet de l'utilisation ou de l'invention telles que définies ci-dessus, on peut citer ceux sélectionnés parmi les éléments du groupe constitué par les compositions d'alkylpolyglycosides et notamment les alkylpolyglucosides et le alkylpolyxylosides, les compositions d'alkylpolyglycosides et d'alcools gras, les esters de polyglycérols et notamment l'oléate de décaglycérol, l'isostéarate de décaglycérol, le monolaurate de décaglycérol, le mono-linoléate de décaglycérol, le mono-myristate de décaglycérol, les esters de polyglycérols alcoxylés, les polyhydroxystéarates de polyglycols, les polyhydroxystéarates de polyglycérols, les polyhydroxystéarates de polyglycérols alcoxylés.

Comme exemples d'huiles optionnellement présents dans la formulation topique cosmétique ou pharmaceutique objet de l'utilisation ou de l'invention telles que définies ci-dessus, on peut citer :
les alcanes linéaires comportant de onze à dix-neuf atomes de carbone, comme l'undécane, le dodécane, le tridécane, le tétradécane, le pentadécane, l'hexadécane, l'heptadécane, l'octadécane et le nonadécane;
les alcanes ramifiés, comportant de sept à quarante atomes de carbone, comme l'isododécane, l'isopentadécane, l'isohexadécane, l'isoheptadécane, l'isooctadécane, l'isononadécane ou l'isoeicosane), ou des mélanges de certains d'entre eux comme ceux cités ci-après et identifiés par leur nom INCI : C₇₋₈ isoparaffin, C₈₋₉ isoparaffin, C₉₋₁₁ isoparaffin, C₉₋₁₂ isoparaffin, C₉₋₁₃ isoparaffin, C₉₋₁₄ isoparaffin, C₉₋₁₆ isoparaffin, C₁₀₋₁₁ isoparaffin, C₁₀₋₁₂ isoparaffin, C₁₀₋₁₃ isoparaffin, C₁₁₋₁₂ isoparaffin, C₁₁₋₁₃ isoparaffin, C₁₁₋₁₄ isoparaffin, C₁₂₋₁₄ isoparaffin, C₁₂₋₂₀ isoparaffin, C₁₃₋₁₄ isoparaffin, C₁₃₋₁₆ isoparaffin ;
les cyclo-alcanes optionnellement substitués par un ou plusieurs radicaux alkyles linéaires ou ramifiés ;
les huiles blanches minérales, comme celles commercialisées sous les noms suivants : MARCOL^{™} 52, MARCOL^{™} 82, DRAKEOL^{™} 6VR, EOLANE^{™} 130 et EOLANE^{™} 150 ;
l'hémisqualane (ou 2,6,10-trimethyl- dodécane ; numéro CAS : 3891-98-3), le squalane (ou 2,6,10,15,19,23-hexamethyltetracosane), le polyisobutène hydrogéné ou le polydécène hydrogéné ;
les mélanges d'alcanes comportant de 15 à 19 atomes de carbone, lesdits alcanes étant des alcanes linéaires, des alcanes ramifiés et des cyclo-alcanes, et plus particulièrement le mélange (M₁) qui comprend pour 100% de sa masse, une proportion massique en alcanes ramifiés supérieure ou égale à 90% et inférieure ou égale à 100% ; une proportion massique en alcanes linéaires supérieure ou égale à 0% et inférieure ou égale à 9%, et plus particulièrement inférieure à 5% et une proportion massique en cyclo-alcanes supérieure ou égale à 0% et inférieure ou égale à 1%, par exemple les mélanges commercialisés sous les noms EMOGREEN^{™} L15 ou EMOGREEN ^{™} L19 ;
les éthers d'alcool gras de formule Z1-O-Z2, dans laquelle Z1 et Z2, identiques ou différents, représentent un radical alkyle linéaire ou ramifié comportant de cinq à dix-huit atomes de carbone, par exemple les dioctyl éther, didécyl éther, didodécyl éther, dodécyl octyl éther, dihexadécyl éther, (1,3-diméthyl butyl) tétradécyl éther, (1,3-diméthyl butyl) hexadécyl éther, le bis(1,3-diméthyl butyl) éther ou le dihexyl éther ;
les mono-esters d'acides gras et d'alcools de formule R'1(C=O)-O-R'2 dans laquelle R'1(C=O)- représente un radical acyle, saturé ou insaturé, linéaire ou ramifié, comportant de huit à vingt-quatre atomes de carbone et R'2 représente, indépendamment de R'1, une chaîne hydrocarbonée saturée ou insaturée, linéaire ou ramifiée comportant de un à vingt-quatre atomes de carbone, par exemple les laurate de méthyle, laurate d'éthyle, laurate de propyle, laurate d'isopropyle, laurate de butyle, laurate de 2-butyle, laurate d'hexyle, cocoate de méthyle, cocoate d'éthyle, cocoate de propyle, cocoate d'isopropyle, cocoate de butyle, cocoate de 2-butyle, cocoate d'hexyle, myristate de méthyle, myristate d'éthyle, myristate de propyle, le myristate d'isopropyle, le myristate de butyle, le myristate de 2-butyle, le myristate d'hexyle, le myristate d'octyle, le palmitate de méthyle, le palmitate d'éthyle, le palmitate de propyle, le palmitate d'isopropyle, le palmitate de butyle, le palmitate de 2-butyle, le palmitate d'hexyle, le palmitate d'octyle , l'oléate de méthyle, l' oléate d'éthyle, l'oléate de propyle, l'oléate d'isopropyle, l'oléate de butyle, l'oléate de 2-butyle, l'oléate d'hexyle, l'oléate d'octyle, le stéarate de méthyle, le stéarate d'éthyle, le stéarate de propyle, le stéarate d'isopropyle, le stéarate de butyle, le stéarate de 2-butyle, le stéarate d'hexyle, le stéarate d'octyle, l'isostéarate de méthyle, l'isostéarate d'éthyle, l'isostéarate de propyle, l'isostéarate d'isopropyle, l'isostéarate de butyle, l'isostéarate de 2-butyle, l'isostéarate d'hexyle, ou l'isostéarate d'isostéaryle ;
les di-esters d'acides gras et de glycérol de formule R'3-(C=O)-O-CH2-CH(OH)-CH2-O-(C=O)-R'4 et de formule R'5-(C=O)-O-CH2-CH(O-(C=O)-R'6)-CH2-OH dans lesquelles R'3-(C=O), R'4-(C=O), R'5-(C=O), R'6-(C=O), identiques ou différents, représentent un groupement acyle, saturé ou insaturé, linéaire ou ramifié, comportant de huit à vingt-quatre atomes de carbone ;
les tri-esters d'acides gras et de glycérol de formule R'7-(C=O)-O-CH2-CH(O-(C=O)-R"8)-CH2-O-(C=O)-R"9, dans laquelle R'7-(C=O), R'8-(C=O) et R'9-(C=O), identiques ou différents, représentent un groupement acyle, saturé ou insaturé, linéaire ou ramifié, comportant de huit à vingt-quatre atomes de carbone.
les huiles végétales, telles que le phytosqualane ou les huiles d'amandes douces, de coprah, de ricin, de jojoba, d'olive, de colza, d'arachide, de tournesol, de germes de blé, de germes de maïs, de soja, de coton, de luzerne, de pavot, de potiron, d'onagre, de millet, d'orge, de seigle, de carthame, de bancoulier, de passiflore, de noisette, de palme, de noyau d'abricot, de calophyllum, de sisymbrium, d'avocat, calendula ou les huiles issues de fleurs ou de légumes ;
les huiles végétales éthoxylées.

Dans la présente invention, on désigne par "cire", un composé ou un mélange de composés insolubles dans l'eau, se présentant sous un aspect solide à une température supérieure ou égale à 45°C. Comme exemples de cires optionnellement présents dans la formulation topique cosmétique ou pharmaceutique objet de l'utilisation ou de l'invention telles que définies ci-dessus, on peut citer les cires d'abeille, de carnauba, de candelilla, d'ouricoury, du Japon, de fibre de liège, de canne à sucre, de paraffines, de lignite, de lanoline, d'ozokérite, polyéthylène ou de silicone ; les cires végétales ou microcristallines, les alcools gras, les acides gras et les glycérides solides à température ambiante comme les beurre de karité ou de caco.

Comme exemples d'eaux thermales et minérales naturelles optionnellement présents dans la formulation topique cosmétique ou pharmaceutique objet de l'utilisation ou de l'invention telles que définies ci-dessus, on peut citer les eaux d'Avène, de Vittel, d'Uriage, de la Roche-Posay, de la Bourboule, d'Enghien-les-Bains, de Saint-Gervais-les bains, de Néris-les-Bains, d'Allevard-les-bains, de Digne, des Maizières, de Neyrac, de Lons le Saunier, de Rochefort, de Saint-Christau, des Fumades, de Tercis-les-Bains, de Bagnères-de-Bigorre, d'Eugenie-les-Bains, l'eau de Challes-les-Eaux, de Volvic, de Vais, de Vernière, d'Aix les Bains, d'Alet, des Abatilles, d'Arcens, d'Arvie, d'Asperjoc, de Badoit, de Cilaos, de Contrexéville, d'Evian, d'Hépar, de Jouvence, du Mont-Roucous, d'Ogeu, d'Orezza, de Parot, de Perrier, de Plancoët, de Quézac, de Rozana, de Saint-Alban-Les-Eaux, de Saint-Amand-les Eaux, de Saint-Georges, de Saint-Géron, de Sainte-Marguerite, de Saint-Yorre, de la Salvetat, de Teissières-lès-Bouliès, de Thonon, Treignac, de Courmayeur, de San Benedetto, de San Pellegrino ou bassin de Vichy.

Comme exemples d'agents déodorants optionnellement présents dans la formulation topique cosmétique ou pharmaceutique objet de l'utilisation ou de l'invention telles que définies ci-dessus, on peut citer les silicates alcalins, les sels de zinc comme les sulfate gluconate, chlorure ou lactate de zinc ; les sels d'ammonium quaternaires comme les sels de cétyltriméthylammonium, les sels de cétylpyridinium ; les dérivés du glycérol comme les caprate ou caprylate de glycérol, le caprate de polyglycérol, le capryloyl glycine, le 1,2 décanediol, le 1,3 propanediol, l'acide salicylique, le bicarbonate de sodium, les cyclodextrines, les zéolithes métalliques, le TRICLOSAN^{™} ; les sels d'aluminium comme les bromhydrate, les chlorhydrate, chlorure, sulfate ou lactate d'aluminium , les chlorhydrates mixtes d'aluminium et de zirconium comme les chlorhydrate, trichlorhydrate, tétrachlorhydrate, pentachlorhydrate ou octochlorhydrate d'aluminium et de zirconium ; le lactate de sodium et d'aluminium ; les complexes de chlorhydrates d'aluminium et de diols comme les complexes chlorhydrate d'aluminium-glycol ou -propylèneglycol, le complexe dichlorhydrate d'aluminium-propylène glycol, le complexe de sesquichlorhydrate d'aluminium-propylène glycol, le complexe chlorhydrate d'aluminium-polyéthylène glycol, le complexe dichlorhydrate d'aluminium-polyéthylène glycol ou le complexe sesquichlorhydrate d'aluminium-polyéthylène glycol.

Comme agents hydrotropes optionnellement présents dans la formulation topique cosmétique ou pharmaceutique objet de l'utilisation ou de l'invention telles que définies ci-dessus, on peut citer les xylènes sulfonates, les cumènes sulfonates, l'hexylpolyglucoside, le 2-éthylhexylpolyglucoside ou le n-heptylpolyglucoside.

Comme agents anti-oxydants optionnellement présents dans la formulation topique cosmétique ou pharmaceutique objet de l'utilisation ou de l'invention telles que définies ci-dessus, on peut citer l'EDTA (acide éthylenediamine tétra-acétique) et ses sels, l'acide citrique, l'acide tartarique, l'acide oxalique, le BHA (butylhydroxyanisol), le BHT (butylhydroxytoluène), les dérivés de tocophérol tels que l'acétate de tocophérol, la DISSOLVINE^{™} GL 47S ou le CONTACTICEL^{™}.

Comme exemples de filtres solaires optionnellement présents dans la formulation topique cosmétique ou pharmaceutique objet de l'utilisation ou de l'invention telles que définies ci-dessus, on peut citer tous ceux figurant dans la directive cosmétique 76/768/CEE modifiée annexe VII

Comme exemples d'agents solubilisant de filtres organiques optionnellement présents dans la formulation topique cosmétique ou pharmaceutique objet de l'utilisation ou de l'invention telles que définies ci-dessus, on peut citer le LANOL^{™} 37T, le DUB^{™} 810PGM, le DUB^{™} DIS, le DUB^{™}DIPA, le DUB^{™} DNPG ou le DUB^{™} SYNERSOL.

Comme exemple d'écran solaire minéral optionnellement présents dans la formulation topique cosmétique ou pharmaceutique objet de l'utilisation ou de l'invention telles que définies ci-dessus, on peut citer les oxydes de titane, de zinc, de cérium, de zirconium, de fer jaune, rouge ou noir, ou de chrome.

Comme exemples de solvants et de co-solvants optionnellement présents dans la formulation topique cosmétique ou pharmaceutique objet de l'utilisation ou de l'invention telles que définies ci-dessus, on peut citer le glycérol, le diglycérol, les oligomères du glycérol, l'éthylène glycol, le propylène glycol, le butylène glycol, l'hexylène glycol, le diéthylène glycol, le xylitol, l'érythritol, le sorbitol, les alcools hydrosolubles tels que l'éthanol, l'isopropanol ou le butanol, les mélanges d'eau et desdits solvants organiques, le propylène carbonate, l'acétate d'éthyle, l'alcool benzylique, le diméthylsulfoxyde (DMSO).

Comme exemples d'agents d'amélioration de la pénétration cutanée optionnellement présents dans la formulation topique cosmétique ou pharmaceutique objet de l'utilisation ou de l'invention telles que définies ci-dessus, on peut citer les glycolséthers comme l'éthylène glycol monométhyl éther, l'éthylène glycol monoéthyl éther, l'éthylène glycol monopropyl éther, l'éthylène glycol monoisopropyl éther, l'éthylène glycol monobutyl éther, l'éthylene glycol monophényl éther, l'éthylène glycol monobenzyl éther, le diéthylène glycol monométhyl éther, le diéthylène glycol monoéthyl éther et le diéthylène glycol mono-n-butyl éther, le diéthylène glycol monoéthyléther (ou Transcutol-P), les acides gras comme l'acide oléique, les esters de glycérol d'acide gras comme le béhénate de glycérol, le palmitostéarate de glycérol, le béhénoyl macroglycérides, le polyoxyéthylène-2-stéaryl éther, le polyoxyéthylène-2-oléyl éthers, des terpènes comme le D-Limonène, des huiles essentielles comme l'huile essentielle d'eucalyptus.

Comme exemples d'agents stabilisants optionnellement présents dans la formulation topique cosmétique ou pharmaceutique objet de l'utilisation ou de l'invention telles que définies ci-dessus, on peut citer les cires microcristallines, et plus particulièrement l'ozokérite, les sels minéraux tels que le chlorure de sodium ou le chlorure de magnésium, les polymères siliconés tels que les copolymères polysiloxane polyalkyl polyéther.

Comme exemples de principes actifs optionnellement présents dans la formulation topique cosmétique ou pharmaceutique objet de l'utilisation ou de l'invention telles que définies ci-dessus, on peut citer :
les vitamines et leurs dérivés, notamment leurs esters, tels que le rétinol (vitamine A) et ses esters (palmitate de rétinyle par exemple), l'acide ascorbique (vitamine C) et ses esters, les dérives de sucre de l'acide ascorbique (comme l'ascorbyl glucoside), le tocophérol (vitamine E) et ses esters (comme l'acétate de tocophérol), les vitamines B3 ou B10 (niacinamide et ses dérivés) ;
les composés montrant une action éclaircissante ou dépigmentante de la peau comme le w-undecelynoyl phénylalanine commercialisé sous l'appellation SEPIWHITE^{™}MSH, le SEPICALM^{™}VG, le mono ester ou le diester de glycérol du w-undecelynoyl phénylalanine, les ω-undecelynoyl dipeptides, l'arbutine, l'acide kojique, l'hydroquinone ;
les composés montrant une action apaisante notamment le SEPICALM^{™} S, l'allantoïne et le bisabolol;
les agents analgésiques ou anti-inflammatoires comme l'acétaminophène, l'aspirine, l'acide salicylique, les salicylate de méthyle, de choline ou de glycol, le 1-menthol, le camphre, l'acide méfénamique, 1'acide fluphénamique, l'indométhacine, l'acide protizidique, le fentiazac, le tolmetin, le phénylbutazone, l'oxyphenbutazone, le clofézone, le pentazocine, le mépirizole, l'hydrocortisone, la cortisone, la dexaméthasone, la fluocinolone, la triamcinolone, la médrysone, la prednisolone, la flurandrénolide, le prednisone, l'halcinonide, le méthylprednisolone, le fludrocortisone, la corticostérone, la paraméthasone, la betaméthasone ; les acide (hétéro)aryl acétique ou 2-(hétéro)aryl propionique comme le diclofénac, l'acide tiaprofènique, l'alminoprofène, l'étodolac, le flurbiprofène, l'ibuprofène, le kétoprofène ou le naproxène.
les agents antiseptiques comme la cetrimide, la povidone-iodine, la chlorhexidine, la viodine, le chlorure de benzalkonium, l'acide benzoïque, le nitrofurazone, le peroxyde de benzoyle, le peroxyde d'hydrogène, l'hexachlorophène, le phénol, le résorcinol ou le chlorure de cétylpyridinium ;
les agents insecticides comme le trichlorfone, le triflumérone, le fenthion, le bendiocarbe, la cyromazine, le dislubenzurone, le dicyclanile, le fluazurone, l'amitraze, la deltaméthrine, la cyperméthrine, le chlorfenbinphose, la fluméthrine, la ivermectine, l'abermectine, l'avermectine, la doramectine, la moxidectine, la zeticyperméthrine, la diazinone, la spinosade, l'imidaclopride, le nitenpyrane, le pyriproxysene, le sipronil, le cythioate, la lufénurone, la selamectine, la milbemycine oxime, le chlorpyrifose, le coumaphose, le propetamphose, Valpha-cyperméthrine, l'highciscyperméthrine, ivermectine, la diflubenzurone, le cyclodiène, le carbamate and ou la benzoyl urée.
les agents antimicrobiens comme les sulfonamides, la néomycine, le tobramycine, le gentamycine, l'amikacine, le kanamycine, le spectinomycine, le paromomycine, le netilmicine, les polypeptides, les céphalosporines ou les oxazolidinones comme la ciprofloxacine, la levofloxacine ou l'ofloxacine ;
les composés montrant une action hydratante comme l'urée, les hydroxy urées, le glycérol, les polyglycérols, le glycérolglucoside, le diglycérolglucoside, les polyglycérylglucosides ou le xylitylglucoside ;
les extraits végétaux riches en polyphénols comme les extraits de raisin, les extraits de pin, les extraits de vin, les extraits d'olives ;
les composés montrant une action amincissante ou lipolytique comme la caféine ou ses dérivés, l'ADIPOSLIM^{™}, l'ADIPOLESS^{™}, la fucoxanthine ;
les protéines N-acylées, les peptides N-acylés comme le MATRIXIL^{™}, les acides aminés N-acylés, les hydrolysâts partiels de protéines N-acylés, les acides aminés, les peptides, les hydrolysâts totaux de protéines,
Les extraits de végétaux, comme les extraits de soja, par exemple la Raffermine^{™}, les extraits de blé par exemple la TENSINE^{™} ou la GLIADINE^{™}, les extraits végétaux, tels que les extraits végétaux riches en tanins, les extraits végétaux riches en isoflavones ou les extraits végétaux riches en terpènes ; les extraits d'algues d'eau douce ou marines ; les extraits de plantes marines ; les extraits marins en général comme les coraux ;
les cires essentielles ;
les extraits bactériens ;
les céramides ;
les phospholipides ;
les composés montrant une action antimicrobienne ou une action purifiante, comme le LIPACIDE^{™} C8G, le LIPACIDE^{™} UG, le SEPICONTROL^{™} A5 ; l'OCTOPIROX^{™} ou le SENSIVA^{™} SC50 ;
les composés montrant une propriété énergisante ou stimulante comme le
PHYSIOGENYL^{™},
le panthénol et ses dérivés comme le SEPICAP^{™} MP ;
Les actifs anti-âge comme le SEPILIFT^{™} DPHP, le LIPACIDE^{™} PVB, le SEPIVINOL^{™}, le SEPIVITAL^{™}, le MANOLIVA^{™}, le PHYTO-AGE^{™}, le TIMECODE"" ; le SURVICODE^{™} ;
les actifs anti-photo vieillissement, les actifs protecteurs de l'intégrité de la jonction dermo-épidermique, les actifs augmentant la synthèse des composants de la matrice extracellulaire comme le collagène, les élastines, les glycosaminoglycanes ;
les actifs agissant favorablement sur la communication cellulaire chimique comme les cytokines ou physiques comme les intégrines ;
les actifs créant une sensation de « chauffe » sur la peau comme les activateurs de la microcirculation cutanée (comme les dérivés de l'acide nicotinique) ou des produits créant une sensation de « fraîcheur » sur la peau (comme le menthol et des dérivés) ;
les actifs améliorant la microcirculation cutanée, par exemple les veinotoniques ;
les actifs drainants, les actifs à visée décongestionnante comme les extraits de ginko biloba, de lierre, de marron d'inde, de bambou, de ruscus, de petit houx, de centalla asiatica, de fucus, de romarin, de saule ;
les agents destinés au traitement des cheveux ou des poils, par exemple des agents protecteurs des mélanocytes du follicule pileux, les agents mimétiques de l'activité de la DOPAchrome tautomérase, les molécules synthétiques mimétiques de la SOD par exemples les complexes de manganèse, des composés antioxydants par exemple les dérivés de cyclodextrine, des composés silicés dérivés d'acide ascorbique, de la pyrrolidone carboxylate de lysine ou d'arginine, des associations de mono- et diester d'acide cinnamique et de vitamine C ;
les agents de bronzage ou de brunissement de la peau, par exemple la dihydroxyacétone (DHA), l'érythrulose, l'aldéhyde mésotartrique, le glutaraldéhyde, le glycéraldéhyde, l'alloxane, la ninhydrine, les extraits végétaux par exemple les extraits de bois rouges du genre Pterocarpus et du genre Baphia comme le Pteropcarpus santalinus, le Pterocarpus osun, le Pterocarpus soyauxii, le Pterocarpus erinaceus, le Pterocarpus indicus ou le Baphia nitida ;
les agents connus pour leur action de facilitation ou d'accélération du bronzage ou du brunissement de la peau humaine, ou pour leur action de coloration de la peau humaine, par exemple les caraténoïdes ( et plus particulièrement le beta carotène et le gamma carotène), les produits commercialisés sous le nom de marque « Carrot oil » et « SunTan Accelerator^{™} »,v« Zymo Tan Complex », « MelanoBronze^{™} », « Monk's pepper extract », « Unipertan VEG-24/242/2002, « », « Try-Excell^{™} », «Actibronze^{™} », « Tyrostan^{™} », « Tyrosinol », « InstaBronze^{™} », « Tyrosilane », « Exymol » »Bronzing SF Peptide powder », « Melitane », « Melatimes Solutions^{™} », « Tanositol^{™} », « Thalitan^{™} , « Phycosaccharide^{™} AG », « Melactiva^{™} » et « Biotanning^{™}».
Comme substances parfumantes ou aromatisantes optionnellement présentes dans la formulation topique cosmétique ou pharmaceutique objet de l'utilisation ou de l'invention telles que définies ci-dessus, on peut citer celles extraites de fleurs comme la rose, le jasmin, la tubéreuse, la champaca, le mimosa, l'œillet, l'osmanthe, la narcisse, la lavande le gardénia ou les fleurs de frangipanier, d'Ylang-ylang, de lotus, d'acacia, d'oranger doux, d'oranger amer ou néroli ; celles extraites de feuilles, de mousse, d'écorce, de résine ou de bourgeons comme les bourgeons de cassis ; les mousses de chêne, de hêtre ou le lichen ; les feuilles d'acacia, de basilic, de valériane, de gentiane, de violette, de géranium, de labdanum, de romarin, de patchouli ou de verveine ; les écorces de cannelle, de frêne, de cassia ou de cascarille ; les bois de santal, de cèdre, de palissandre, d'aloès, de bouleau, de gaïac ; les baumes du Pérou ou du tolu ; la résine de Benjoin, la myrrhe, la résine de labdanum, la résine d'élémi, l'oliban, l'opoponax, le guggul, celles extraites d'aiguilles et de branches de pin ou d'épicéa ou de sapin ; celles extraites de l'estragon, du lemon grass, de la sauge ou du thym ; celles extraites de gousses, de fèves ou de baies comme la fève de tonka, les gousses de vanille, la cardamome, la coriandre, la badiane, l'amande amère, le cumin, les clous de girofle, les baies de genièvre ; oucelles extraites des agrumes comme le citron, l'orange dont la linette et la bergamote, la mandarine ; celles extraites des racines comme les racines d'angélique, de céleri, de cardamone, d'iris, d'accore, de cactus ou de vétyver.

Comme substances parfumantes ou aromatisantes optionnellement présentes dans la formulation topique cosmétique ou pharmaceutique objet de l'utilisation ou de l'invention telles que définies ci-dessus, on peut aussi citer celles d'achillée millefeuille, d'accore calamus, d'ail, d'ajowan, d'amyris, d'aneth, d'anis, d'angélique, d'arbre à thé, de basilic, de bay saint thomas, de benjoin de bergamote, de bois de Gaïac, de bois de Hô, de bois de rose, de bois de santal, de bois de siam, de bouleau noir, de camomille, de camphrier, de cannelle, de cardamone, de carotte, de carvi, de cèdre, de céleri, de christe marine, de ciste, de citron, de citronnelle, de clémentine, l'huile de combawa, de copahu, de coriandre, de cryptomeria, de cumin, de curcuma, de cyprès, d'encens, l'huile d'épinette, d'estragon, de fenouil, de fragonia, de galbanum, de gaulthérie (wintergreen), de genévrier, de géranium, de gingembre, de clou ou de feuille de girofle, d'helichryse, d'hysope, d'iary, d'inule, de katrafay, de Khella, de kunzea, de lavande, de lavandin, de mandarine, de niaouli, de menthe poivrée, d'orange, de pamplemousse, de romarin, de thym, d'Ylang Ylang, de ravintsara, de sauge, de Cabreuva, de Lemongrass, de Palmarosa, de millepertuis, de jasmin, de camomille, de mélisse, de pin, de gingembre, de persil, d'armoise, de chanvre, de houblon, ou encore de serpolet.

Comme substances parfumantes ou aromatisantes optionnellement présentes dans la formulation topique cosmétique ou pharmaceutique objet de l'utilisation ou de l'invention telles que définies ci-dessus, on peut encore citer le musc, le castoreum, la civette, l'ambre gris, l'absolue de cire d'abeille, l'hyraceum.

Comme composés chimiques substances parfumantes ou aromatisantes synthétiques optionnellement présentes dans la formulation topique cosmétique ou pharmaceutique objet de l'utilisation ou de l'invention telles que définies ci-dessus, on peut citer :
des hydrocarbures terpéniques (mono et sesquiterpènes) tels que l'alpha ou le béta myrcène, le limonène, l'alpha ou le béta pinène, le camphène, le cadinène, le cedrène, le farnesène, le caryophyllène, le chamazulène, 1,1-diméthoxy-2,2,5-triméthyl-4-hexène, le curcumène, le crythmène, les himachelènes, le limonène ou le paracymène ; les oxydes de rose ou de tétranorlabdane ; les terpinènes, les terpinolènes ou les vetivènes ;
des esters tels que les acétates de benzyle, de bornyle, de citronellyle, de cédryle, de dihydromyrcényle, de diméthyl-benzylcarbinyle, d'éthyle, de farnésyle, de fenchyle, d'hexyle, de géranyle, d'isobutyle, d'isononyle, d'isopentyle, d'isobornyle, d'isopulégyle, de linalyle, de menthyle, de méthyl phényl carbinyle, de néryle, de nonyle, de 2-(tert-butyl) cyclohexyle, de phényléthyle, de 4-(tert-butyl) cyclohexyle, de prényle, de styrallyle, de terpènyle ou de vetyvéryle ; l'anthralinate de methyle, les benzoates de benzyle, d'isobutyle ou de linalyle, la coumarine, les butanoates d'éthyle, de benzyle ou d'isoamyle, les butyrates de benzyle, d'éthyle, d'isoamyle, ou de lynalyle ; les cinnamates de butyle, d'allyle ou d'éthyle, les formiates de benzyle, de citronellyle, d'hédione, de geranyle, de méthyle ; le glycinate d'éthylméthylphényle, le glycolate d'allyl-amyle, l'heptanoate d'allyle, l'isobutyrate de phénoxyéthyle, l'isobutyrate de cis-3-hexényle, le méthacrylate d'isoamyle, le naphtolate d'ethyle, le néopentanoate d'hexyle, les propionates d'amyle, d'alkylcyclohexyle, d'allylcyclohexane, de lynalyle, de styralyle ou de citronellyle ; les salicylates de méthyle, de benzyle ou d'éthyle ou le tiglate d'hexyle ;
des alcools et des phénols tels que l'alcool benzoïque, l'alpha terpinéol, l'anéthol, le carotol, le chavicol, l'estragol , le cinéol, l'alcool cinnamique, le citronellol, le p-crésol, l'alcool cumique, le 3,7-diméthyl-1-octanol, le diméthyle benzyle carbinol, l'alcool fenchylique, le l'eucalyptol, le farnesol, l'eugénol, l'alcool isononylique, l'isoeugénol, le gaïacol, le géraniol, le globutol, le linanol, le menthol, le dihydromyrcénol, le nérolidol, le nérol, l'alcool phenyl éthylique, le safrol, l'isosafrol, le phytol, l'iso phytol, le terpineol, le tétrahydrolinalol, le tétrahydromyrcénol, le thymol, le vétivérol ou l'undécavertol ;
des aldéhydes tels que les aldéhydes phényl acétique, salicylique, anisique, caprylique, cinnamique ou hexyl cinnamique ; le bourgeonal, le citral, le citronellal, l'hydroxy-citronellal, le citronellyloxyacétaldéhyde, le cyclamènaldéhyde, le cuminaldéhyde, le cyclal, le 2,4-diméthyl-3-cyclohexène-1-carboxaldéhyde, le dodécanal, l'éthanal, l'octanal, le décanal, les géranials, l'hélional, les lactones telles que les gamma-undécalactones, le lilial, le méthyl n-nonyl acétaldéhyde, le méthyl octyl acétaldéhyde, l'undécanal ou la vanilline ;
des cétones tels que la benzyl acétone, la 7-méthyl-2H-benzo-1,5-dioxepin-3(4H)-one, la carvone, le camphre, la civéttone, les damascones, les damascénones, l'éthyl-amyl-cétone, l'éthyl-hexyl-cétone, la géranyl cétone, la jasmone, les irones, le 3-hydroxy-2-methyl-4H-pyran-4-one, l'éthyl maltol, la menthone, l'isomenthone, la muscone, la méthyl hepténone, les ionones comme la méthyl-ionone, la 4-méthyl acétophénone, la méthylpentylcétone, la méthylheptylcétone, la méthylhexylcétone, l'alpha-isométhylionone, ou la méthylcédrylcétone ;
des éthers tels que l'anéthol, le benzyléthléther, le cédryl methyl ether, le p-crésyl méthyl éther ;
des muscs artificiels issus de différents composés nitrés, les musc ambrette, les musc cétones, les musc xylène, les musc macrocycliques,
des nitriles tels que les triméthyl-3 , 5, 7-octane (ène) nitriles et leurs dérivés alpha-substitués, le citronellyl nitrile, le citronitrile, le géranyl-nitrile.

Les exemples suivants illustrent l'invention sans toutefois la modifier.

### Préparation d'un copolymère Tulipalin A-acide itaconique (ratio molaire RA= 2), réticulé par du tétraacrylate de pentaérythritol (ratio molaire RB = 0,0040) selon l'invention (P1).

30,0 g de tert-butanol et 1,5 g d'eau déminéralisée sont versés dans un réacteur sous agitation mécanique. 6,50 g (0,05 mole) d'acide itaconique, 4,91 g (0,05 mole) de Tulipalin A et 0,11 g (0,0003 mole) de tétraacrylate de pentaérythritol y sont ensuite ajoutés. La dispersion obtenue est désoxygénée par barbotage d'azote pendant cinquante minutes puis chauffée à 80°C. 0,20 g de peroxyde de dilauroyle y est ensuite ajouté et le tout est maintenu à 80°C sous agitation mécanique pendant trois heures. 4,91 g (0,05 mole) de Tulipalin A sont alors ajoutés (pour atteindre le ratio molaire RA = 2) suivis de 0,11 g (0,0003 mole) de tétraacrylate de pentaérythritol (pour atteindre le ratio molaire RB = 0,0040) et de 0,20 g de peroxyde de dilauroyle. Le milieu obtenu est maintenu à 80°C sous agitation mécanique pendant trois nouvelles heures. Il est ensuite refroidi à 35°C et filtré sur papier et le précipité recueilli est finalement séché dans une étuve à 50°C sous un vide de 20 mbars. 13,78 g du copolymère attendu (P1) sont ainsi isolés, ce qui correspond à un rendement final de 83,4%.

### Préparation d'un copolymère Tulipalin A-acide itaconique (RA = 3), réticulé par du tétraacrylate de pentaérythritol (RB = 0,0039) selon l'invention (P2)

30,0 g de tert-butanol et 1,5 g d'eau déminéralisée sont versés dans un réacteur sous agitation mécanique. 4,88 g (0,0375 mole) d'acide itaconique, 5,30 g (0,054 mole) de Tulipalin A et 0,10 g (0,0002838 mole) de tétracrylate de pentaérythritol y sont ensuite ajoutés. La dispersion obtenue est désoxygénée par barbotage d'azote pendant 45 minutes puis ensuite chauffée à 80°C. 0,18 g de peroxyde de dilauroyle y est alors ajouté et le tout est maintenu est maintenu à 80°C sous agitation mécanique pendant trois heures. 5,30 g (0,054 mole) de Tulipalin A sont alors ajoutés (pour atteindre le ratio molaire RA = 3), suivi de 0,10 g (0,0002838 mole) de tétraacrylate de pentaérythritol (pour atteindre un ratio molaire RB = 0,0039) et 0,18 g de peroxyde de dilauroyle sont de nouveau rajoutés au milieu réactionnel hétérogène. Le milieu obtenu est maintenu à 80°C sous agitation mécanique pendant trois nouvelles heures. Il est ensuite refroidi à 35°C et filtré sur papier et le précipité recueilli est finalement séché dans une étuve à 50°C sous un vide de 20 mbars. 12,29 g du copolymère (P2) sont ainsi isolés, ce qui correspond à un rendement final de 78,4%.

### Préparation d'un copolymère de Tulipalin A-acide itaconique (RA = 0,4) réticulé par du tétraacrylate de pentaérythritol (RB = 0,0069) selon l'invention (P3)

30,0 g de tert-butanol et 1,5 g d'eau déminéralisée sont versés dans un réacteur sous agitation mécanique. 9,30 g (0,0715 mole) d'acide itaconique, 1,40 g (0,001425 mole) de Tulipalin A et 0,09 g (0,0002572 mole) de tétraacrylate de pentaérythritol y sont ensuite ajoutés. La dispersion obtenue est désoxygénée par barbotage d'azote pendant 60 minutes puis ensuite chauffée à 80°C. 0,17 g de peroxyde de dilauroyle y est alors ajouté et le tout est maintenu est maintenu à 80°C sous agitation mécanique pendant trois heures. 1,40 g (0,01425 mole) de Tulipalin A sont alors ajoutés (pour atteindre le ratio molaire RA = 0,4), suivi de 0,09 g (0,0002572 mole) de tétraacrylate de pentaérythritol (pour atteindre un ratio molaire RB = 0,0069) et 0,17 g de peroxyde de dilauroyle sont de nouveau rajoutés au milieu réactionnel hétérogène. Le milieu obtenu est maintenu à 80°C sous agitation mécanique pendant trois nouvelles heures. Il est ensuite refroidi à 35°C et filtré sur papier et le précipité recueilli est finalement séché dans une étuve à 50°C sous un vide de 20 mbars. 3,32 g du copolymère (P3) sont ainsi isolés, ce qui correspond à un rendement final de 27%.

### Préparation d'un copolymère de Tulipalin A-acide itaconique (RA = 1) réticulé par du tétraacrylate de pentaérythritol (RB = 0,004) selon l'invention (P4)

50,0 g d'eau déminéralisée sont versés dans un réacteur sous agitation mécanique. 6,50 g (0,05 mole) d'acide itaconique, 1,39 g d'une solution de soude à 48% massique (0,00167 mole) pour solubiliser partiellement l'acide itaconique 4,91 g (0,05 mole) de Tulipalin A et 0,1057 g (0,0003 mole) de tétraacrylate de pentaérythritol y sont ajoutés. La dispersion obtenue est désoxygénée par barbotage d'azote pendant 40 minutes puis ensuite chauffée à 80°C. 0,1356 g de 2,2'azobis(2-méthylpropanamidine) y est alors ajouté et le tout est maintenu est maintenu à 80°C sous agitation mécanique pendant trois heures. Il est ensuite refroidi à 35°C et filtré sur papier et le précipité recueilli est finalement séché dans une étuve à 50°C sous un vide de 20 mbars. 4,77 g du copolymère (P4) sont ainsi isolés, ce qui correspond à un rendement final de 41,4%.

### Préparation d'un copolymère Tulipalin A-acide itaconique (RA = 2) et réticulé par du triacrylate de triméthylol propane (RB = 0,004) selon l'invention (P5)

30,0 g de tert-butanol et 1,5 g d'eau déminéralisée sont versés dans un réacteur sous agitation mécanique. 6,50 g (0,05 mole) d'acide itaconique, 4,91 g (0,05 mole) de Tulipalin A et 0,1057 g (0,0003 mole) de triacrylate de triméthylol propane y sont ensuite ajoutés. La dispersion obtenue est désoxygénée par barbotage d'azote pendant 45 minutes puis ensuite chauffée à 80°C. 0,20 g de peroxyde de dilauroyle y est alors ajouté et le tout est maintenu est maintenu à 80°C sous agitation mécanique pendant trois heures. 4,91 g (0,05 mole) de Tulipalin A sont alors ajoutés (pour atteindre le ratio molaire RA = 2), suivi de 0,1057 g (0,0003 mole) de triacrylate de triméthylol propane (pour atteindre un ratio molaire RB = 0,004) et 0,20 g de peroxyde de dilauroyle sont de nouveau rajoutés au milieu réactionnel hétérogène. Le milieu obtenu est maintenu à 80°C sous agitation mécanique pendant trois nouvelles heures. Il est ensuite refroidi à 35°C et filtré sur papier et le précipité recueilli est finalement séché dans une étuve à 50°C sous un vide de 20 mbars. 13,01 g du copolymère (P5) sont ainsi isolés, ce qui correspond à un rendement final de 79%.

### Préparation d'un copolymère Tulipalin A-acide itaconique (RA = 2) selon l'invention (P6)

30,0 g de tert-butanol et 1,5 g d'eau déminéralisée sont versés dans un réacteur sous agitation mécanique. 6,50 g (0,05 mole) d'acide itaconique et 4,91 g (0,05 mole) de Tulipalin A y sont ensuite ajoutés. La dispersion obtenue est désoxygénée par barbotage d'azote pendant 50 minutes puis ensuite chauffée à 80°C. 0,20 g de peroxyde de dilauroyle y est alors ajouté et le tout est maintenu est maintenu à 80°C sous agitation mécanique pendant trois heures. 4,91 g (0,05 mole) de Tulipalin A sont alors ajoutés (pour atteindre le ratio molaire RA = 2) et 0,20 g de peroxyde de dilauroyle sont de nouveau rajoutés au milieu réactionnel hétérogène. Le milieu obtenu est maintenu à 80°C sous agitation mécanique pendant trois nouvelles heures. Il est ensuite refroidi à 35°C et filtré sur papier et le précipité recueilli est finalement séché dans une étuve à 50°C sous un vide de 20 mbars. 12,36 g du copolymère (P6) sont ainsi isolés, ce qui correspond à un rendement final de 75%.

### Préparation d'un copolymère Tulipalin A (la)-acide itaconique (RA = 2) réalisé avec recyclage du filtrat selon l'invention (P7)

16,0 g du filtrat d'un essai de copolymérisation du Tuliplain A et de l'acide Itaconique (RA = 1 ; avec ajout du Tulipalin en une seule fois) dans le tert-butanol sont placés dans le réacteur (ce filtrat contenant de l'acide itaconique n'ayant pas réagi avec le Tulipalin A lors d'une précédente synthèse). 2,45 g (0,025 mole) de Tulipalin A sont ajoutés. La dispersion obtenue est désoxygénée par barbotage d'azote pendant 50 minutes puis chauffée à 80°C. 0,10 g de peroxyde de dilauroyle y est alors ajouté et le tout est maintenu à 80°C sous agitation mécanique pendant 6 heures. Le milieu réactionnel est ensuite refroidi à 35°C et filtré sur papier et le précipité recueilli est finalement séché dans une étuve à 50°C sous un vide de 20 mbars. 2,80 g de copolymère (P7) sont isolés correspondant à un rendement final de 48,6%.

### Préparation d'un copolymère Tulipalin A-acide itaconique (RA = 1) réticulé par du tétraacrylate de pentaérythritol (RB = 0,003) selon l'invention (P8)

30,0 g de tert-butanol et 1,5 g d'eau déminéralisée sont versés dans un réacteur sous agitation mécanique. 6,5 g (0,05 mole) d'acide itaconique, 4,9 g (0,05 mole) de Tulipalin A et 0,11 g (0,0003 mole) de tétracrylate de pentaérythritol y sont ajoutés. Une solution homogène est obtenue. Cette solution est désoxygénée par barbotage d'azote pendant 45 minutes puis chauffée à 80°C. 0,2 g de péroxyde de dilauroyle y est alors ajouté et le tout est maintenu à 80°C sous agitation mécanique pendant 3 heures. Le milieu réactionnel est ensuite refroidi à 35°C et filtré sur papier et le précipité recueilli est finalement séché dans une étuve à 50°C sous un vide de 20 mbars. 5,63 g de copolymère (P8) sont isolés correspondant à un rendement final de 48,9%.

### Préparation d'un copolymère de Tulipalin A-acide itaconique (RA = 1) réticulé par du tétraacrylate de pentaérythritol (RB = 0,003) selon l'invention (P9)

50,0 g de tertio-butanol et 1,5 g d'eau déminéralisée sont versés dans un réacteur sous agitation mécanique. 13,0 g (0,1 mole) d'acide itaconique, 9,8 g (0,1 mole) de Tulipalin A (soit un ratio molaire RA = 1) et 0,21 g (0,0006 mole) de tétracrylate de pentaérythritol y sont ajoutés. Une suspension est obtenue. Cette suspension est désoxygénée par barbotage d'azote pendant 45 minutes puis chauffée à 56°C. 0,4 g de peroxyde de dilauroyle y est alors ajouté et le tout est maintenu à 56°C sous agitation mécanique pendant 3 heures. Le milieu réactionnel est ensuite refroidi à 25°C et filtré sur papier et le précipité recueilli est finalement séché dans une étuve à 50°C sous un vide de 20 mbars. 5,63 g de copolymère (P9) sont isolés correspondant à un rendement final de 28,7%.

### Préparation d'un homopolymère de Tulipalin (la) réticulé par du tétraacrylate de pentaérythritol (P10) (hors invention)

30,0 g de tert-butanol et 1,5 g d'eau déminéralisée sont versés dans un réacteur sous agitation mécanique. 9,81 g (0,1 mole) de Tulipalin A et 0,1057 g (0,0003 mole) de tétracrylate de pentaérythritol y sont ajoutés. Une solution homogène est obtenue. Cette solution est désoxygénée par barbotage d'azote pendant 60 minutes puis chauffée à 80°C. 0,20 g de péroxyde de dilauroyle y est alors ajouté et le tout est maintenu à 80°C sous agitation mécanique pendant 3 heures. Le milieu réactionnel est ensuite refroidi à 35°C et filtré sur papier et le précipité recueilli est finalement séché dans une étuve à 50°C sous un vide de 20 mbars. 9,87 g de copolymère (P10) sont isolés. Ce polymère n'est pas soluble (même après ajout NaOH pour ajuster le pH à 6-8) dans l'eau et ne présente donc pas de propriétés épaississantes de phases aqueuses.

### Préparation hors invention d'un homopolymère d'acide itaconique (IIa) réticulé par du tétraacrylate de pentaérythritol (P11) (hors invention)

Le même procédé que celui décrit pour l'homopolymère (P10) a été mis en œuvre en remplaçant le 30,0 g tert-butanol par 50,0 g l'acétone et les 9,81 g de Tulipalin A par 13,0 g d'acide itaconqiue. 13,2 g de copolymère (P11) sont isolés après séchage du milieu réactionnel, de qui correspond à un rendement de 100,0%. Ce polymère est soluble dans l'eau après ajout de soude pour ajustement du pH à 6-8 mais ne présente pas de propriétés épaississantes de phases aqueuses.

### Préparation d'un copolymère Tulipalin A-acide itaconique (ratio molaire RA = 0,2), réticulé par du tétraacrylate de pentaérythritol (ratio molaire RB = 0,0055) (P12) (hors invention)

60,0 g de tert-butanol et 3,05 g d'eau déminéralisée sont versés dans un réacteur sous agitation mécanique. 21,68 g (0,16667 mole) d'acide itaconique, 1,63 g (0,01667 mole) de Tulipalin A et 0,194 g (0,00055 mole) de tétracrylate de pentaérythritol y sont ensuite ajoutés. La dispersion obtenue est désoxygénée par barbotage d'azote pendant 45 minutes puis ensuite chauffée à 80°C. 0,365 g de peroxyde de dilauroyle y est alors ajouté et le tout est maintenu est maintenu à 80°C sous agitation mécanique pendant trois heures. 1,63 g (0,01667 mole) de Tulipalin A sont alors ajoutés (pour atteindre le ratio molaire RA = 0,2), suivi de 0,194 g (0,00055 mole) de tétraacrylate de pentaérythritol (pour atteindre un ratio molaire RB = 0,0055) et 0,365 g de peroxyde de dilauroyle sont de nouveau rajoutés au milieu réactionnel hétérogène. Le milieu obtenu est maintenu à 80°C sous agitation mécanique pendant trois nouvelles heures. Il est ensuite refroidi à 35°C et filtré sur papier et le précipité recueilli est finalement séché dans une étuve à 50°C sous un vide de 20 mbars. 3,91 g du copolymère (P5) sont ainsi isolés, ce qui correspond à un rendement final de 15,4%.

### Préparation d'un copolymère de Tulipalin A-acide itaconique (lia) (ratio molaire RA = 4) et réticulé par du tétraacrylate de pentaérythritol (ratio molaire RB = 0,0036) (P13) (hors invention)

30,0 g de tert-butanol et 1,5 g d'eau déminéralisée sont versés dans un réacteur sous agitation mécanique. 2,60 g (0,02 mole) d'acide itaconique, 3,92 g (0,04 mole) de Tulipalin A et 0,063 g (0,00018 mole) de tétraacrylate de pentaérythritol y sont ensuite ajoutés. Une dispersion est obtenue. La dispersion obtenue est désoxygénée par barbotage d'azote pendant cinquante minutes puis chauffée à 80°C. 0,12 g de peroxyde de dilauroyle y est ensuite ajouté et le tout est maintenu à 80°C sous agitation mécanique pendant trois heures. 3,92 g (0,04 mole) de Tulipalin A sont alors ajoutés (pour atteindre le ratio molaire RA = 4) suivis de 0,063 g (0,00018 mole) de tétraacrylate de pentaérythritol (pour atteindre le ratio molaire RB = 0,0040) et de 0,12 g de peroxyde de dilauroyle. Le milieu obtenu est maintenu à 80°C sous agitation mécanique pendant trois nouvelles heures. Il est ensuite refroidi à 35°C et filtré sur papier et le précipité recueilli est finalement séché dans une étuve à 50°C sous un vide de 20 mbars. 8,9 g du copolymère attendu (P13) sont ainsi isolés, ce qui correspond à un rendement final de 84%.

Evaluation des propriétés épaississantes des polymères préparés. Les propriétés épaississantes de copolymères à base de Tulipalin A et d'acide itaconique selon l'invention (P1), (P2), (P3), (P4),(P5), (P6), (P7), (P8) et (P9) sont consignés dans le tableau 1 suivant. Elles sont comparées à celles des polymères hors invention (P10), (P11), (P12) et (P13). Les gels sont préparés en dispersant le polymère dans l'eau et en ajustant si nécessaire le pH à une valeur supérieure ou égale à 6 et inférieure ou égale à 8 avec de la soude.

**[Tableau 1]**

| Viscosité de gels aqueux de polymère (en mPa.s) Brookfield LVT (Mobile 4 ; Vitesse 6 | | | |
|---|---|---|---|
| Polymère selon l'invention | Proportions massiques en polymère dans le gel | Sans NaCl | Avec 01% NaCl |
| P1 | 2% | 14.720 | 8.680 |
| P1 | 5% | > 100.000 | Non déterminé (n.d.) |
| P2 | 2% | 8.840 | 2.560 |
| P2 | 5% | 63.000 | n.d. |
| P3 | 2% | 13.000 | 9.640 |
| P3 | 5% | > 100.000 | 39.400 |
| P4 | 2% | 1.630 | n.d. |
| P4 | 5% | 10.000 | n.d. |
| P5 | 2% | 3.065 | 1.200 |
| P5 | 5% | 37.000 | n.d. |
| P6 | 2% | 1.220 | 860 |
| P6 | 5% | 11.600 | n.d. |
| P7 | 2% | 24.000 | n.d. |
| P7 | 5% | 580 | n.d. |
| P8 | 5% | 43.800 | n.d. |
| P9 | 5% | 34.000 | n.d. |
| Polymère hors | Proportions massiques | | |
| P10 | | n.d. | n.d. |
| P11 | | Non épaississant | n.d. |
| P12 | 2% | 4.040 | 3.180 |
| P12 | 5% | 41.600 | n.d. |
| P13 | 2% | 5 | 5 |
| P13 | 5% | 20 | n.d. |

Exemples de formulation topiques cosmétiques selon l'invention

**[Tableau 2]**

| Formulation moussante aux AHA | |
|---|---|
| Ingrédients | Proportions massiques |
| Eau déminéralisée | qsp 100,0% |
| Glycérine | 2,0% |
| Copolymère (P1) | 1,0% |
| ORAMIX^{™} CG110 | 5,0% |
| Lauryl éther Sulfate de sodium 28% | 6,0% |
| Acide glycolique 70% | 5,0% |
| EUXYL^{™} K712 | 1,0% |
| Triéthanolamine | qs pH4 |

**[Tableau 3]**

| Formulation de protection solaire | |
|---|---|
| Ingrédients | Proportions massiques |
| Eau déminéralisée | qsp 100,00% |
| Copolymère (P1) | 0,50% |
| FLUIDIFEEL^{™} EASY | 3,00% |
| LANOL^{™} 37T | 8,00% |
| EMOGREEN^{™} L19 | 4,00% |
| NEO HELIOPAN^{™} OS | 5,00% |
| NEO HELIOPAN^{™} BMT | 4,00% |
| NEO HELIOPAN^{™} HMS | 5,00% |
| DL alpha tocophérol | 0,05% |
| EUXYL^{™} K903 | 1,00% |
| CONTACTICEL^{™} | 2,00% |
| EPHEMER^{™} | 2,00% |
| TINOSORB^{™} M | 5,00% |
| Acide citrique 25% | 0,04% |

**[Tableau 4]**

| Formulation de protection solaire SPF 50 | |
|---|---|
| Ingrédients | Proportions massiques |
| Eau déminéralisée | qsp 100,0% |
| Glycérine | 3,0% |
| Copolymère (P3) | 0,5% |
| NEO HELIOPAN^{™} HYDRO | 1,5% |
| Triéthanolamine 50% | 1,7% |
| SENSANOV^{™} WR | 3,0% |
| NEO HELIOPAN^{™} OS | 5,0% |
| NEO HELIOPAN^{™} BMT | 6,0% |
| LANOL^{™} 37T | 7,0% |
| DUB^{™} SSIC | 5,0% |
| A15-TiO2-SX-NJE8 | 5,0% |
| DL alpha tocophérol | 0,2% |
| EUXYL^{™} PE9010 | 0,5% |
| SENSIVA^{™} PA40 | 0,5% |

**[Tableau 5]**

| Emulsions de type huile dans eau | | | |
|---|---|---|---|
| Ingrédients | Proportions massiques | | |
| MONTANOV^{™} 68 | 1,0% | 1,0% | 1,0% |
| TRIGLYCERIDES 5545 | 20,0% | 20,0% | 20,0% |
| Eau déminéralisée | qsp 100,0% | | |
| Copolymère (P1) | 0,3% | 0,5% | 1,0% |
| EUXYL^{™} PE9010 | 1,0% | 1,0% | 1,0% |

**[Tableau 6]**

| Emulsions de type huile dans eau avec émollients | | | |
|---|---|---|---|
| Ingrédients | Proportions massiques | | |
| MONTANOV 68 | 3,0% | 3,0% | 3,0% |
| TRIGLYCERIDES 5545 | 20,0% | 20,0% | 25,0% |
| EMOGREEN^{™} L19 | 5,0% | 10,0% | 0,0% |
| EMOGREEN HP40 | 0,0% | 0,0% | 5,0% |
| Eau déminéralisée | qsp 100,0% | | |
| Copolymère (P1) | 1,0% | 0,5% | 1,0% |
| EUXYL PE9010 | 1,0% | 1,0% | 1,0% |

**[Tableau 7]**

| Emulsions de type huile-dans-eau avec huiles végétales | | | | |
|---|---|---|---|---|
| Ingrédients | Proportions massiques | | | |
| MONTANOV^{™} 68 | 3,0% | 3,0% | 3,0% | 3,0% |
| LANOL^{™} 2681 | 0,0% | 0,0% | 0,0% | 0,0% |
| LANOL^{™} 99 | 20,0% | 0,0% | 0,0% | 0,0% |
| Huile d'amandes douces | 0,0% | 30,0% | 0,0% | 0,0% |
| Huile de jojoba | 0,0% | 0,0% | 30,0% | 0,0% |
| Huile d'olive | 0,0% | 0,0% | 0,0% | 30,0% |
| Eau déminéralisée | qsp 100,0% | | | |
| Copolymère (P5) | 0,5% | 1,0% | 1,0% | 1,0% |
| EUXYL^{™} PE9010 | 1,0% | 1,0% | 1,0% | 1,0% |

**[Tableau 8]**

| Emulsions de type huile dans eau | | | | |
|---|---|---|---|---|
| Ingrédients | Quantité massique (%) | | | |
| FLUIDIFEEL^{™} EASY | 3,0% | 3,0% | 3,0% | 3,0% |
| TRIGLYCERIDES 5545 | 20,0% | 20,0% | 10,0% | 10,0% |
| EMOGREEN^{™} L15 | 0,0% | 0,0% | 5,0% | 5,0% |
| Eau déminéralisée | qsp 100,0% | | | |
| Copolymère (P5) | 1,0% | 0,5% | 0,5% | 1,0% |
| EUXYL^{™} PE9010 | 1,0% | 1,0% | 1,0% | 1,0% |

**[Tableau 9]**

| Emulsions de type huile dans eau | | | |
|---|---|---|---|
| Ingrédients | Proportions massiques | | |
| FLUIDIFEEL^{™} EASY | 1,0% | 1,0% | 1,0% |
| TRIGLYCERIDES 5545 | 20,0% | 10,0% | 0,0% |
| EMOGREEN^{™} L15 | 0,0% | 5,0% | 0,0% |
| LANOL^{™} 2681 | 0,0% | 0,0% | 15,0% |
| Eau déminéralisée | qsp 100,0% | | |
| Copolymère (P2) | 0,5% | 0,5% | 1,0% |
| EUXYL^{™} PE9010 | 1,0% | 1,0% | 1,0% |

**[Tableau 10]**

| Emulsion hydratante et anti-âge de type huile dans eau | |
|---|---|
| Ingrédients | Proportions massiques |
| Eau déminéralisée | qsp 100,0% |
| copolymère (P1) | 1,0% |
| FLUIDIFEEL^{™} EASY | 3,0% |
| Huile d'amandes douces | 10,0% |
| LANOL^{™} 2681 | 7,0% |
| DUB ZENOAT^{™} | 3,0% |
| AQUAXYL^{™} | 3,0% |
| APAR'AGE^{™} | 2,0% |
| EUXYL^{™} K903 | 1,0% |
| Parfum | 0,1% |
| Acide lactique | qs pH = 5 |

**[Tableau 13]**

| Ingrédients utilisés dans les formulations topiques mentionnées ci-dessus | |
|---|---|
| Nom commercial | Composition ou nom INCI |
| ORAMIX CG110 | Caprylyl-Capryl Glucoside |
| EUXYL K712 | Sodium Benzoate (and) Potassium Sorbate (and) Aqua |
| FLUIDIFEEL^{™} EASY | Lauryl Glucoside (and) Myristyl Glucoside (and) Poly- |
| LANOL^{™} 37T | Triheptanoin |
| EMOGREEN^{™} L19 | C15-19 Alkane |
| NEO HELIOPAN^{™} OS | Salicylate d'éthylhexyle |
| NEO HELIOPAN^{™} BMT | bis-éthylhexyloxyphenol méthoxyphényl triazine |
| NEO HELIOPAN^{™} HMS | Homosalate |
| EUXYL^{™} K903 | Benzyl Alcohol (and) Benzoic Acid (and) |
| CONTACTICEL^{™} | Aqua (and) Butylene Glycol (and) Hydrolyzed |
| EPHEMER^{™} | Caprylic/Capric Triglyceride - Undaria Pinnatifida |
| TINOSORB^{™} M | Methylene Bis-Benzotriazolyl Tetramethylbutylphenol |
| NEO HELIOPAN^{™} HYDRO | Phenylbenzimidazole Sulfonic Acid |
| SENSANOV^{™} WR | C20-22 Alkyl Phosphate (and) C20-22 Alcohols |
| DUB SSIC^{™} | Isocetyl Stearoyl Stearate |
| A15-TiO2-SX-NJE8 | Titanium Dioxide (and) Silica (and) Jojoba Esters |
| EUXYL^{™} PE9010 | Phenoxyethanol (and) Ethylhexylglycerin |
| SENSIVA^{™} PA40 | Phenylpropanol (and) Propanediol (and) Caprylyl |
| MONTANOV^{™} 68 | Cetearyl Alcohol (and) Cetearyl Glucoside |
| TRIGLYCERIDES 5545 | Caprylic-Capric Triglyceride |
| EMOGREEN^{™} HP40 | C15-19 Alkane (and) Hydrogenated Polyfamesene |
| LANOL^{™} 2681 | Coco-Caprylate-Caprate |
| LANOL^{™} 99 | Isononyl Isononanoate |
| Huile d'amandes douces | Prunus amygdalus dulcis oil |
| Huile de jojoba | Simmondsia chinensis seed oil |
| Huile d'olive | Olea europaea fruit oil |
| EMOGREEN^{™} L15 | C15-19 Alkane |
| DUB ZENOAT^{™} | Propanediol Dicaprylate |
| AQUAXYL^{™} | Xylitylglucoside (and) Anhydroxylitol (and) Xylitol |
| APAR'AGE^{™} | Water (and) Propanediol (and) Asparagopsis Armata |

## Revendications

1. Copolymère linéaire ou réticulé constitué d'unités monomériques issues du 3-méthylène-dihydro-2(3H)-furanone de formule (I) : 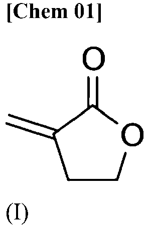
et d'unités monomériques issues de l'acide méthylène-1,4-butanedioïque de formule (II) : 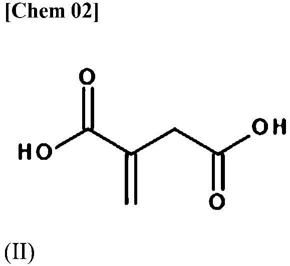
dans lequel le rapport molaire RA ayant comme numérateur le nombre de moles d'unités monomériques issues de composé de formule (I) et comme dénominateur le nombre de moles d'unités monomériques issues du composé de formule (II) est supérieur ou égal à 0,4 et inférieur ou égal à 3,0.

2. Copolymère linéaire ou réticulé selon la revendication 1, dans lequel ledit rapport molaire RA est supérieur ou égal à 1,5 et inférieur ou égal à 2,5.

3. Copolymère réticulé selon l'une quelconque des revendications 1 ou 2 dans lequel le rapport molaire RB ayant comme numérateur le nombre de moles d'unités monomériques issues du monomère de réticulation et comme dénominateur la somme des nombres de moles d'unités monomériques issues du composé de formule (I) et de moles d'unités monomériques issues du composé de formule (II) est supérieur ou égal à 0,0010 et inférieur ou égal à 0,0070.

4. Copolymère réticulé selon la revendication 3, dans lequel ledit rapport molaires RB est supérieur ou égal à 0,0020 et inférieur ou égal à 0,0060.

5. Copolymère réticulé selon l'une quelconque des revendication 3 ou 4, dans lequel les unités monomériques issues du monomère de réticulation, le sont d'un composé choisi dans le groupe constitué par le méthylène bis(acrylamide), le tétraacrylate de pentaérythritol, le triacrylate de triméthylol propane, la triallylamine, le pentaérythritol triallyléther, le diacrylate de 1,6-hexanediol, le diacrylate d'éthylèneglycol et le diallyloxyacétate de sodium (DAOAS).

6. Copolymère réticulé selon la revendication 5, dans lequel les unités monomériques issues du monomère de réticulation, le sont d'un composé choisi dans le groupe constitué par le tétraacrylate de pentaérythritol et le triacrylate de triméthylol propane.

7. Procédé de préparation d'un copolymère linéaire ou réticulé tel que défini à l'une quelconque des revendications 1 à 6 comprenant les étapes successives suivantes :
- une étape a) de mise à disposition :
- d'un solvant choisi dans le groupe comprenant l'eau, l'éthanol, le propanol, l'isopropanol, le butanol, l'isobutanol, les sec-butanol, tert-butanol, l'acétone, la méthyl éthyl cétone, le cyclohexane, l'acétate d'éthyle ou un mélange de deux ou de plusieurs d'entre eux ;
- dudit monomère de formule (II) et dudit monomère de formule (I) dans des proportions molaires telles que le rapport molaire R'A ayant comme numérateur le nombre de moles de composé de formule (I) et comme dénominateur le nombre de moles de composé de formule (II) soit supérieur ou égal à 0,4 et inférieur ou égal à 3,0 ; et
- optionnellement dudit monomère de réticulation choisi dans le groupe constitué par le méthylène bis(Acrylamide), le tétraacrylate de Pentaérythritol, le triacrylate de triméthylol propane, la triallylamine pentaérythritol triallyléther, le diacrylate de 1,6-hexanediol, le diacrylate d'éthylèneglycol et le diallyloxyacétate de sodium, dans des proportions molaires telles que le rapport molaire R'B ayant comme numérateur le nombre de moles de monomère de réticulation et comme dénominateur la somme des nombres de moles de composé de formule (I) et de composé de formule (II), soit supérieur ou égal à 0,001 et inférieur ou égal à 0,007 ;
- une étape b) d'addition de la totalité des moles de composé de formule (II) dans le solvant mis à disposition à l'étape a) pour former une solution ou une dispersion dudit composé de formule (II),
- optionnellement une étape c) de neutralisation partielle ou totale du composé de formula (II), en ajoutant une base dans ladite solution ou dispersion formée à l'étape b), pour former une solution ou une dispersion dudit composé de formule (II) partiellement ou totalement neutralisé ;
- une étape d) d'addition soit d'une partie, soit de la totalité des moles de composé de formule (I) mises à disposition à l'étape a) dans ladite solution ou dispersion formée à l'étape b) ou dans ladite solution ou dispersion formée à l'étape c), pour former une solution ou une dispersion dudit composé de formule (II), optionnellement partiellement ou complètement neutralisé, et dudit composé de formule (I) ;
- optionnellement une étape e) d'addition soit d'une partie, soit de la totalité des moles de monomère de réticulation dans ladite solution ou dispersion obtenue à l'étape d), pour former une solution ou une dispersion dudit composé de formule (II), optionnellement partiellement ou complètement neutralisé, dudit composé de formule (I) et dudit monomère de réticulation ;
- une étape f) de désoxygénation de ladite solution ou dispersion obtenue à l'étape d), respectivement à l'étape optionnelle e), par barbotage en son sein d'un gaz inerte, plus particulièrement d'azote, pendant une durée supérieure ou égale 30 minutes et inférieure ou égale à 90 minutes, pour obtenir une solution ou une dispersion désoxygénée dudit composé de formule (II), optionnellement partiellement ou complètement neutralisé, dudit composé de formule (I) et dudit monomère de réticulation ;
- une étape g) de chauffage à une température supérieure ou égale à 50°C et inférieure ou égale à 95°C de ladite solution ou dispersion obtenue à l'étape f), pour obtenir une solution ou une dispersion désoxygénée et chauffée dudit composé de formule (II), optionnellement partiellement ou complètement neutralisé, dudit composé de formule (I) et dudit monomère de réticulation;
- une étape h) de polymérisation des monomères présents au sein de ladite solution ou dispersion désoxygénée et chauffée obtenue à l'étape g), par addition d'un amorceur de polymérisation, plus particulièrement le peroxyde dilauroyle ou le dichlorhydrate 2,2'-azobis(2-méthylpropionamidine) et maintien à une température de polymérisation supérieure ou égale à 50°C et inférieure ou égale à 95°C, pendant une durée supérieure ou égale à 150 minutes et inférieure ou égale à 300 minutes, pour obtenir un précipité dudit copolymère optionnellement réticulé attendu et optionnellement dudit monomère de formule (II) n'ayant pas réagi au sein dudit solvant mis à disposition à l'étape a) ;
- optionnellement une étape i) d'addition dans ledit solvant obtenu à l'étape h), comprenant le précipité dudit copolymère optionnellement réticulé attendu et optionnellement ledit monomère de formule (II) n'ayant pas réagi, du reste dudit composé de formule (I) et optionnellement du reste dudit monomère de réticulation mis à disposition à l'étape a), puis de polymérisation du reste des monomères présents par addition d'un amorceur de polymérisation, particulièrement le peroxyde dilauroyle ou le dichlorhydrate de 2,2'-azobis(2-méthylpropionamidine) en maintenant la température à une valeur supérieure ou égale à 50°C et inférieure ou égale à 95°C, pendant une durée supérieure ou égale à 150 minutes et inférieure ou égale à 300 minutes, pour obtenir une dispersion dudit copolymère optionnellement réticulé attendu ;
- une étape j) de refroidissement de ladite dispersion dudit copolymère optionnellement réticulé obtenu à l'étape i) jusqu'à une température supérieure ou égale à 15°C et inférieure ou égale à 30°C; et
- soit une étape k) de filtration de ladite dispersion refroidie obtenue à l'étape j), pour recueillir ledit copolymère optionnellement réticulé attendu suivie d'une étape 1) de séchage dudit copolymère optionnellement réticulé recueilli,
- soit une étape m) d'atomisation ladite dispersion refroidie obtenue à l'étape j), pour obtenir ledit copolymère optionnellement réticulé attendu sous forme de poudre.

8. Procédé de préparation selon la revendication 7, dans lequel les étapes b), c) optionnelle, d) et e) optionnelle sont simultanées et constituent une étape B).

9. Procédé de préparation selon l'une des revendications 7 ou 8, dans lequel le solvant mis à disposition à l'étape a) est soit l'eau, soit le tert-butanol soit un mélange tert-butanol-eau.

10. Procédé de préparation selon la revendication 9, comprenant les étapes successives suivantes :
- une étape a) de mise à disposition :
- d'un mélange tert-butanol-eau comprenant pour 100% massique, une proportion massique supérieure ou égale à 90% et inférieure ou égale à 98% massique de tert-butanol et une proportion massique supérieure ou égale à 2% massique et inférieure ou égale à 10% massique d'eau ;
- dudit monomère de formule (II) et dudit monomère de formule (I) dans des proportions molaires telles que le rapport molaire R'A ayant comme numérateur le nombre de moles de composé de formule (I) et comme dénominateur le nombre de moles de composé de formule (II) soit supérieur ou égal à 1,0 et inférieur ou égal à 3,0 ; et optionnellement
- dudit monomère de réticulation choisi dans le groupe constitué par le tétraacrylate de pentaérythritol et le triacrylate de triméthylol propane, dans des proportions molaires telles que le rapport molaire R'B ayant comme numérateur le nombre de moles de monomère de réticulation et comme dénominateur la somme des nombres de moles de composé de formule (I) et de composé de formule (II), soit supérieur ou égal à 0,003 et inférieur ou égal à 0,005 ;
- une étape B) d'addition dans ledit mélange tert-butanol-eau dans le solvant mis à disposition à l'étape a) de la totalité des moles de composé dudit composé de formule (II), de 50% des moles de composé de formule (I) et optionnellement de 50% des moles dudit monomère de réticulation pour former une solution ou une dispersion dudit composé de formule (II), dudit composé de formule (I) et optionnellement dudit monomère de réticulation ;
- une étape f) de désoxygénation de ladite solution ou dispersion obtenue à l'étape B), par barbotage d'azote en son sein, pendant une durée supérieure ou égale 45 minutes et inférieure ou égale à 60 minutes, pour obtenir une solution ou une dispersion désoxygénée dudit composé de formule (II), dudit composé de formule (I) et optionnellement dudit monomère de réticulation ;
- une étape g) de chauffage à une température supérieure ou égale à 75°C et inférieure ou égale à 85°C de ladite solution ou dispersion obtenue à l'étape f), pour obtenir une solution ou une dispersion désoxygénée et chauffée dudit composé de formule (II), dudit composé de formule (I) et optionnellement dudit monomère de réticulation ;
- une étape h) de polymérisation des monomères présents au sein de ladite solution ou dispersion désoxygénée et chauffée obtenue à l'étape g), par addition de peroxyde dilauroyle et maintien à une température de polymérisation supérieure ou égale à 75°C et inférieure ou égale à 85°C, pendant un durée supérieure ou égale à 150 minutes et inférieure ou égale à 200 minutes pour obtenir une précipité dudit copolymère optionnellement réticulé attendu et optionnellement dudit monomère de formule (II) n'ayant pas réagi au sein dudit solvant mis à disposition à l'étape a) et ;
- une étape i) d'addition dans ledit solvant obtenu à l'étape h), comprenant le précipité dudit copolymère optionnellement réticulé attendu et optionnellement ledit monomère de formule (II) n'ayant pas réagi, du reste dudit composé de formule (I) et optionnellement du reste dudit monomère de réticulation mis à disposition à l'étape a), puis de polymérisation du reste des monomères présents par addition de peroxyde dilauroyle et maintien à une température de polymérisation supérieure ou égale à 50°C et inférieure ou égale à 85°C, pendant une durée supérieure ou égale à 150 minutes et inférieure ou égale à 200 minutes, pour obtenir une dispersion dudit copolymère optionnellement réticulé attendu ;
- une étape j) de refroidissement de ladite dispersion dudit copolymère optionnellement réticulé obtenu à l'étape i) jusqu'à une température supérieure ou égale à 15°C et inférieure ou égale à 30°C; et
- soit une étape k) de filtration de ladite dispersion refroidie obtenue à l'étape j), pour recueillir ledit copolymère optionnellement réticulé attendu suivie d'une étape 1) de séchage dudit copolymère optionnellement réticulé recueilli,
- soit une étape m) d'atomisation ladite dispersion refroidie obtenue à l'étape j) pour obtenir ledit copolymère optionnellement réticulé attendu sous forme de poudre.

11. Utilisation du copolymère linéaire ou réticulé tel que défini à l'une quelconque des revendications 1 à 6, en vue d'épaissir, de stabiliser ou d'émulsionner une formulation topique cosmétique ou pharmaceutique ou d'y suspendre des de particules solides.

12. Formulation topique cosmétique ou pharmaceutique **caractérisée en ce qu'**elle comprend pour 100,0% de sa masse, de 0,1% à10,0% et plus particulièrement de 0,5% à 5,0% copolymère linéaire ou réticulé tel que défini à l'une quelconque des revendications 1 à 6, comme agent épaississant, comme agent stabilisant ou comme agent émulsionnant de ladite formulation topique cosmétique ou pharmaceutique ou comme agent apte et destiné à suspendre des particules solides au sein de ladite formulation topique cosmétique ou pharmaceutique.
